# EUROPEAN PATENT APPLICATION

(11) **EP 2 174 585 A1**
(43) Date of publication of application: **14.04.2010**
(21) Application number: 08425634.6
(22) Date of filing: 29.09.2008
(51) Int. Cl.: A61B 5/00

(54) **Olfactory identification test kit for determining neurological disorders**

(71) Applicant: Maremmani, Carlo, 55042 Forte dei Marmi (LU) (IT)
(72) Inventor: Maremmani, Carlo, 55042 Forte dei Marmi (LU) (IT); Rossi, Giuseppe, 54100 Massa (MS) (IT)
(74) Representative: Perani, Aurelio

(57) **Abstract**

The invention relates to a kit comprising a set of 25 to 45 testers and a directions for use of the kit, wherein each tester comprises a single odorant, other than those of the other testers, adhered to an appropriate odorless substrate common to all testers, each tester carries a tester identification mark, all testers have the same format, colors, size and texture feel, the only distinctive aspect thereamong being said identification mark, which uniquely corresponds to the single odorant adhered to each tester, and the area delimited by the odorant on each tester, as well as its color and position on the substrate are common to all testers. Such kit is disposable, practical and handy, is suitable for mass screening, provides reliable results and is easy to use.

## Description

The present invention relates to an olfactory identification test kit for determining neurological disorders.

Olfactory disorders are frequent in general population. A full loss of such sense (anosmia) is found in 1% of general population in the USA (see references #31-34). Recent epidemiological surveys suggest that 5% of general population suffers from a significant reduction in the sense of smell (hyposmia) (see references #35-36) and a higher percent is found in higher age ranges (see references #37).

Subjects with smell dysfunction are often unaware of their impairment in this sense (see references #38-44). Certain neurodegenerative diseases such as Parkinson's disease involve a significant smell impairment, which can be assessed by special tests even in the earliest stages of this disease. Clinical and pathoanatomical considerations suggest that this sensory disorder precedes the onset of the typical motor symptoms of the disease, wherefore a smell disorder might be used as a pre-clinical marker of Parkinson's disease and/or other neurodegenerative disorders (see references #45-65).

Therefore, smell quantification tests may be of help in the diagnosis and treatment of impairments of this sense and may be further useful in assessing important neurological diseases in man, while still in the subclinical stage.

The olfactory system allows man to sense the presence of osmically active molecules in the environment and to perceive the intensity and type of odor, and possibly to identify it.

During the last 50 years, tests have been developed for assessing smell function. The following 29 tests are known in the literature (Table 1):

**Table 1**

| Test name | | Publication year |
|---|---|---|
| Venous olfactory test. | | |
| | 1. (1A) Kamio T. Methods of olfactory test, expecially clinical studies on the methods of venous olfactory test. Nippon Jibiinkoka Gakkai Kaiho 1963; 66: 469-85. (1B) Keyaki Y. The clinical studies on the method of venous olfactory test. I. The modified method of venous olfactory test. II. On the effect which the diluited olfactory substance in the course of venous olfactory test brings on the latent time. Nippon Jibiinkoka Gakkai Kaiho 1964 sep; 67: 1320-38. (1C) Saito Y. Studies of olfactory sensation curves in a venous olfactory test. Nippon Jibiinkoka Gakkai Kaiho 1966; 69: 1833 - 912. | 1963 -1966 |
| Olfactory test using standard odorous substances. | | 1975 |
| | 2. Shiokawa H. The clinical studies on the olfactory test using standard odorous substances. The change of olfactory acuity due to ageing. Nippon Jibiinkoka Gakkai Kaiho. 1975 20; 78: 1258-70. | |
| A standard olfactory acuity test. | | |
| | 3. Oyaizu S. A clinical study of the diagnosis of olfactory disturbance by means of a standard olfactory acuity test. Nippon Jibiinkoka Gakkai Kaiho. 1978 aug 20; 81(8): 780-8. | 1978 |
| Olfactory testing: rules for odor identification. | | |
| | 4. Cain WS, Krause RJ. Olfactory testing: rules for odor identification. Neurol Res. 1979; 1(1): 1-9. | 1979 |
| The pyridine scale. | | |
| | 5. Sherman AH, Amoore JE, Weigel V. The piridine scale for clinical measurement of olfactory threshold: a quantitative reevaluation. Otolaryngol Head Neck Surg. 1979 nov-dec; 87(6):717-33. | 1979 |
| T&T olfactometry. | | |
| | 6. Zusho H. Olfactometry in Japan. 0337 / 931431 in Bari, Italy. 281-85. | 1983 |
| Connecticut Chemosensory Clinical Research Center Test (CCCRC test). | | |
| | 7. Cain WS, Gent J, Catalanotto FA, Goodspeed RB. Clinical evaluation of olfaction. Am J Otolaryngol. 1983; 4: 252-6. | 1983 |
| University of Pennsylvania Smell Identification Test - UPSIT. | | |
| | 8. Doty RL, Shaman P, Dann M. Development of Pennsylvania Smell Identification Test: a standardized microencapsulated test of olfactory function. Physiol Behav 1984; 32: 489-502. | 1984 |
| Odorant Confusion Matrix. | | |
| | 9. Wright HN. Characterization of olfaction dysfunction. Arch Otolaryngol Head Neck Surg. 1987 feb; 113(2): 163-8. | 1987 |
| Dutch odor identification test (GITU). | | 1988 |
| | 10. Hendriks AP. Olfactory dysfunction. Rhinology 1988; 26: 229-51. | |
| The intravenous olfaction test, the Alinamin ® | | |
| | 11. Furukawa M, Kamide M, Miwa T, Umeada R. Significance of intravenous olfaction test using thiamine propyldisulfide (Alinamin) in olfactometry. Auris Nasus Larynx 1988; 15: 25-31. | 1988 |
| A quantitative olfactory function test for the Italian population: standardization and normative data. | | |
| | 12. Maremmani C, Nocita G, Rossi G, Bonuccelli U, Muratorio A. Un test quantitativo per la funzione olfattiva: standardizzazione e dati normativi. Nuova Riv di Neurologia, aprile 1993; vol 3 n° 2: 58-64. | 1993 |
| San Diego Odor Identification Test (SDOIT). | | |
| | 13. Murphy C, Anderson JA, Markinson S. Psychophysical assessment of chemosensory disorders in clinical populations: In: Kurihara K, Suzuki N, Ogawa H, eds. Olfaction and Taste XI. Tokyo, Japan: Springer-Verlag Tokyo; 1994: 609-13. | 1994 |
| | Cross-Cultural Smell Identification Test (CC-SIT). | |
| | 14. Doty RL, Marcus A, Lee WW. Development of the 12-item Cross-Cultural Smell Identification Test (CC-SIT). Laryngoscope 1996; 106: 353-356. | 1996 |
| Match-to-sample odorant discrimination task (MODT) for children and adolescents. | | |
| | 15. Richman RA, Sheele PR, Wallace K, Hyde JM, Coplan J. Olfactory performance during childhood. II. Developing a discrimination task for children. J Pediatr. 1995 sept; 127(3): 421-6. | 1995 |
| Combined Olfactory Test (COT). | | |
| | 16. Validation of a combined olfactory test. Robson AK, Woollons AC, Horrocks C, Williams S, Dawes PJD. Clin Otolaryngol 1996; 21 (6): 512-18. | 1996 |
| Sniffin Sticks. | | 1996 |
| | 17. Kobal G, Hummel T, Sekinger B, Barz S, Roscher S, Wolf S. "Sniffin' Sticks": Screening of olfactory performance. Rhinology 1996; 34: 222-6. | |
| The alcohol sniff test (AST). | | |
| | 18. Davidson TM, Murphy C. Rapid clinical evaluation of anosmia. The alcohol sniff test. Arch Otolaryngol Head Neck Surg. 1997 jun; 123(6): 591-4. | 1997 |
| Scandinavian Odor-Identification Test (SOIT). | | |
| | 19.Nordin S, Brämerson A, Lidén E, Bende M. The Scandinavian Odor-Identification Test: development, reliability, validity and normative data. Acta Otolaryngol 1998; 118: 226-34. | 1998 |
| The spray test. | | |
| | 20. Kremer B, Klimek L, Mosges R. Clinical validation of a new olfactory test. Eur Arch Otorhinolarryngol. 1998; 225(7): 355-8. | 1998 |
| Jet Stream Olfactometer (JSO). | | |
| | 21. Ikeda K, Tabata K, Oshima T et al. Unilateral examination of olfactory threshold using test stream olfactometer. Auris Nasal Larynx. 1999; 26: 435-439. | 1999 |
| Smell diskettes test. | | |
| | 22. Simmen D, Briner HR, Hess K. Screening of olfaction with smell diskettes. Laryngorhinootologie 1999; 78: 125-30. | 1999 |
| A threshold like measure: the random-test. | | |
| | 23.Kobal G, Palisch K, Wolf SR, Meyer ED, Huttenbrink KB, Roscher S, Wagner R, Hummel T. A threshold-like measure for the assessment of olfactory sensitivity: the "random" procedure. Eur Arch Otorhinolaryngol. 2001 may; 258(4): 168-72. | 2001 |
| Olfactory test - Biolfa(R). | | |
| | 24. Lancau JB, Faulcon P, Werner A, Bonfilis P. Normative data of the Biolfa(R) olfactory test. Ann Otolaryngol Chir Cervicofac. 2002; 119(3): 164-9. | 2002 |
| Clinical assessment of retronasal olfactory function. | | |
| | 25.Heilmann S, Strehle G, Rosenheim K, Damm M, Hummel T. Clinical assessment of retronasal olfactory function. Arch Otolaryngol Head Neck Surg 2002; 128: 414-8. | 2002 |
| Odor stick identification test for Japanese (OSIT-J). | | |
| | 26. Saito S, Ayabe S, Naito N, Gotow N, Kobayakawa T, Mise M, Takashima Y. Development of an odor identification test for Japanese people; verification of stick type and card type. J Jpn Assoc Odor Environ 2003; 34: 1-6. | 2003 |
| European test of olfactory capabilities (ETOC). | | |
| | 27.Development of the ETOC: a European test of olfactory capabilities. Thomas-Danguin T, Rouby C, Sicard G, Vigouroux M, Farget V, Johanson A, Bengtzon A, Hall G, Ormel W, De Graaf C, Rousseau F, Dumont JP. Rhinology. 2003 Sep;41(3):142-51 | 2003 |
| The sniff magnitude test (SMT). | | |
| | 28.Frank RA, Dulay MF, Gesteland RC. Assessment of the Sniff Magnitude Test as a clinical test of olfactory function. Physiol Behav. 2003; 78(2): 195-204. | 2003 |
| Barcellona Smell Test - 24 (BAST-24). | | |
| | 29. Cardesin A, Alobid I, Benitez P, Sierra E, de Haro J, Bernal-Sprekelsen M, Picado C, Mllol J. Barcellona Smell Test - 24 (BAST-24): validation and smell characteristics in the healthy Spanish population. Rhinology 2006 mar; 44(1): 83-9. | 2006 |

Only a few of these tests have been validated in a large population sample and have been employed for clinical neurological studies. Tests having these features are: UPSIT (#8 of Table 1), standardized olfactory test for the Italian population (#12 of Table
1) and the "sniffing sticks" (#17 of Table 1).

The main methods for assessing olfactory function may be substantially divided into the following 3 basic groups:
1. Tests for evaluating the odor detecting threshold. This method is used to determine the minimum concentration at which the odorants may be perceived by the subjects.
2. Odor discriminating tests. This method assesses the ability to discriminate different odors.
3. Odor identification tests. The subject is required to assign a name to the proposed odor (the odor is much above the detection threshold). In recent years these tests have been developed as multiple-choice or forced-choice tests. Here, the subject is required to select the right choice from possible options.
   Certain olfactory tests may be combinations of multiple smell tests, such as a smell threshold test + an odor discriminating test + and an odor identification test (e.g. Sniffing Sticks) (#17 of Table 1). Other tests only include two of the three basic methods (e.g. Connecticut Chemosensory Clinical Research Center Test - CCCRC test; Combined Olfactory Test - COT; Olfactory test - Biolfa®)(#7; 16; 24 of Table 1).

In humans, the sense of smell may have both quantitative (a) and qualitative (b) dysfunctions:
a) quantitative dysfunctions: an odor may be perceived less than in healthy individuals (hyposmia) or not perceived at all at concentrations that usually allow strong perception of such odor in healthy individuals (anosmia);
b) qualitative dysfunctions: the perception of one or more odors is altered and does not correspond to the correct perception of that odor/s (dysosmia). Clinical studies teach that a subject may have a totally distorted perception of smells, to cases in which a patient may perceive, for example, the odor of coffee as similar to excrements or vice versa. This olfactory impairment may be obviously increasingly graded, to conditions that can only be detected by special smell tests.

In recent years, the term hyposmia has been generally employed to indicate a hypofunction of the sensory system, of the quantitative type (hyposmia proper) the qualitative type (dysosmia), or a combination of both.

The tests based on smell thresholds, defined as minimum concentrations at which a person can perceive the presence of an odor, are not suitable for clinical use for a number of reasons:
1) as mentioned above, a subject with smell dysfunction may perceive odors in an altered/distorted manner (dysosmia proper). Smell threshold tests cannot assess this kind of dysfunction (dysosmia), that is highly frequent in certain degenerative neurological diseases (see references #81-84). With the smell threshold method, odors have to be administered at such low concentrations that even a normal subject could not identify them. On a smell threshold basis, what can be perceived is a sensation other than fresh air, but the odor cannot be identified. If reference is made to the auditory system, this test might be considered like a theoretical auditory threshold test using words (and not pure tones); at very low volume, the subject can perceive a sound, without understanding the word message; something similar occurs with smell threshold tests.
2) in a smell threshold test, the subject being examined is required to smell glass jars (flasks) containing a transparent liquid and to indicate which flasks contain an odorant and which ones contain double-distilled water (all flasks contain the same volume of liquid, but at least 5 flasks are provided for each odorant dilution, two of them containing the odorant solution, and three containing doubled-distilled water only). The subject starts to smell the flasks with higher dilutions (more diluted odorant) and, if he/she cannot perceive the smell, he successively smells the sets of flasks with lower dilutions (more concentrated odorant). The subject is required to identify which flasks contain the odor. This method is strongly affected by any olfactory "contamination" such as: minor residues of osmically active molecules of the detergents employed for glass washing (jars and flasks) and/or for washing the work environment in which the test is carried out; development of microscopic mold colonies on the glass and/or the work environment, etc.) Even the slightest contamination with any material that has a smell makes the test wholly unreliable. Large and olfactorily "aseptic" environments are not easy to find in clinical facilities, in which there is a prevailing requirement of maintaining the environments clean or even bacteriologically sterile, but smells are widely present, due to the use of detergents for everyday cleaning and to the presence of disinfecting solutions and the like;
3) smell threshold tests are of complex preparation, subject to degradation within a few hours, require long administration times and are also strongly influenced by attention skills, that are required to a high degree in the subject under examination;
4) smell threshold tests often provide very different results from one subject to another and sometimes even in one subject examined at different times.

Due to the above reasons, smell threshold tests are ill suited for use in clinical practice. Nonetheless, it should be noted that the results obtained by this type of test have been very useful for diagnosis of selective anosmias and crucial for formulating theories about olfactory signal perception and translation: a) primary smells; b) specific smell receptors (for classes of odor molecules) present on dendrites of olfactory cells (see references #66-75). Hypothesis and theories have been extensively demonstrated with time (see reference #76).

Odor discrimination tests assess the skill of a person to perceive more or less similar smells as different. These are useful tests, whose further development and clinical use are desirable.

The sense of smell allows perception, identification and classification of a given smell. From this point of view, odor identification tests are theoretically an excellent option. Nonetheless, in practice, for many years attempts have been made to overcome two major obstacles of this method.

A first problem is that, in humans, the ability to identify an odor out of its formal context, e.g. the odor of coffee out of the context of a classical cup and/or of any other assistance (color, particular circumstance), that might help to identify it as a hot aromatic beverage to be consumed early in the morning "to wake up", etc., is an incredibly difficult test for people that have no particular skill therefor.

In a study published in Lancet in 1962, Sumner (see reference #77) clearly showed that healthy subjects do not easily identify common odors, such as chocolate, lemon, anise, etc.; even coffee could not be identified in almost 20% of the cases. The author also showed that the odors that were used for smell assessment during neurological examinations were often unsuitable for the purpose, i.e. were even less recognizable than others by healthy subjects. Thus, Sumner suggested to use at least the odorants that were found to be less difficult to identify, with the hope of obtaining better information about the function of the olfactory system. Nonetheless, the author concluded that this type of odor identification tests provided no additional information besides the simple patient answer: "I can/cannot smell it". That kind of odor identification test could onlu essentially demonstrate a very serious alteration of the olfactory system (i.e. "I cannot smell odors"), providing rather rough information. Less severe or slight smell impairment levels could not be ascertained because the odor identification request led to no concrete information, as both patients and healthy individuals made a considerable number of errors.

An important improvement in odor identification tests was achieved when it was found that, by asking a subject to identify odors, while submitting a number of possible options for each odor, with only one right answer, the subject was able to recognize and identify all or almost all odors (see references #85-87).

Multiple-choice odor identification tests have been developed ever since. These psycho-physiological tests have been found to provide reliable quantitative data in test-retest contexts, i.e. the scores obtained upon first administration (test) are substantially coincident with the scores obtained upon second test administration (retest) to the same subjects, after a variable period of about three months from the first administration. These tests can detect even minor defects of this sensory system, even when the subject is unaware of his/her impairment in this chemical sense (see references #8, 12, 39, 81, 88-90).

A second problem, which affects proper measurement of the olfactory function by odor identification tests, is of cultural nature. Not all odors are known in the same manner in different populations. Therefore, an odor identification test well suited to US populations, such as the University of Pennsylvania Smell Identification Test (see reference #8) provides no satisfactory results in Italy, because odors such as "shunk", "pumpkin pie", *"root beer" "wintergreen"* (gaultheria procumbens) etc., can be easily identified by people who live in the United States, but European, and more particularly Italian people are not able to identify them. Furthermore, odors that are culturally present in both Italy and U.S. (peach, garlic, banana, cloves, coconut, pineapple) are identified in highly different percentages in both populations (see reference #12).

The possibility of developing an odor identification test suitable for all populations is in contrast with the possibility of employing a large number of odorants belonging to the real cultural background of the populations for which the test is designed. Tests that address all populations, such as the Cross-Cultural Smell Identification Test (CC-SIT) (see reference #14), contains 12 odorants only. An olfactory test with such small number of odors has a poor sensitivity (see reference #77) and excludes aromas/odors that are much more significant for specific cultural contexts (the following will be excluded in Italy: coffee, mushroom, apple, lavender, orange, sage, rosemary, basil, etc.) Therefore, any hypothesis of universal olfactory odor identification tests have been criticized (see reference #77) and, in practice, olfactory odor identification tests are usually being developed for each country/geographical area, in which the most familiar odors for resident populations are used.

The need of preparing odor identification tests in response to the cultural contexts of the place in which such tests are used has been often expressed in the literature (see references #12, 77-79). This minimizes cultural difficulties in aromatic substance recognition, as well as non-olfactory interferences from test results, and provide apparent reliability advantages.

This requirement has led to the development of region-specific odor identification tests, such as the *University of Pennsylvanya Smell Identification Test* (UPSIT) (see reference #8); the Quantitative olfactory function test for the Italian population (see reference #12); the *Scandinavian Odor Identification Test* (SOIT)" (see reference #19); the *Odor Stick Identification Test for Japanese* (OSIT-J) (see reference #26); the *Barcellona Smell Test - 24* (BAST-24) (see reference #29).

Particularly, the publication by Maremmani et al., dated 1993 (see reference #12) describes a method for a forced, multiple choice odor identification test, suited for the Italian population. This test has been used for surveys on neurodegenerative neurological diseases. This method proposed the use of 40 odorants belonging to the Italian cultural background. Each odorant was diluted to appropriate concentrations and was contained in a numbered amber-colored glass vial. In spite of its effectiveness, the test was found to be bulky, heavy, fragile, unhandy and certainly unsuitable for low-cost home delivery without incurring in the risk of damages. Furthermore, that test also had the drawback that it had to be prepared anew after a few days because, upon contact with air and microbiological agents, odors changed and degraded, generating distorted olfactory perceptions. Finally, the test was not designed to be disposable, the same containers being used for several persons; this involved microbiological pollution, as well as the transmission of germs.

Therefore, the object of the present invention is to provide an odor identification test suitable for the Italian population, that is disposable, practical and handy, and that is suitable for mass screening, provides reliable results and is easy to use.

This object has been fulfilled by an assessment kit as defined in claim 1. Further advantages and embodiments of the invention are set out in the dependent claims.

Thus, the invention relates to a kit comprising a set of testers and directions for use. Further features and advantages of the invention will become readily apparent from the following detailed description, referring to exemplary and non-limiting embodiments of the invention and to the annexed drawings, in which:
- Figure 1 shows an embodiment of the kit of the invention;
- Figure 2 is a graphical representation of an embodiment of the directions for use of the kit of the invention;
- Figure 3 is a diagram that represents the number of errors detected in female subjects divided into age ranges;
- Figure 4 is a diagram that represents the number of errors detected in male subjects divided into age ranges;
- Figure 5 is a diagram that represents the ratio of the number of errors to the age of the female subjects;
- Figure 6 is a diagram that represents the ratio of the number of errors to the age of the male subjects;
- Figure 7 is a diagram that represents a comparison between the results obtained from the group of controls, both male and female, and the results obtained from the group of Parkinson's disease patients, both male and female, in the 50 to 59 age range;
- Figure 8 is a diagram that represents a comparison between the results obtained from the group of controls, both male and female, and the results obtained from the group of Parkinson's disease patients, both male and female, in the 60 to 69 age range;
- Figure 9 is a diagram that represents a comparison between the results obtained from the group of controls, both male and female, and the results obtained from the group of Parkinson's disease patients, both male and female, in the 70 to 79 age range;
- Figure 10 is a diagram that represents a comparison between the number of errors obtained from the administration of the kit of the invention to a number of female controls, and the number of errors obtained from the administration of the same kit to a group of female patients suffering from Parkinson's disease, in the 50 to 59 age range;
- Figure 11 is a diagram that represents a comparison between the number of errors obtained from the administration of the kit of the invention to a number of female controls, and the number of errors obtained from the administration of the same kit to a group of female patients suffering from Parkinson's disease, in the 60 to 69 age range;
- Figure 12 is a diagram that represents a comparison between the number of errors obtained from the administration of the kit of the invention to a number of female controls, and the number of errors obtained from the administration of the same kit to a group of female patients suffering from Parkinson's disease, in the 70 to 79 age range;
- Figure 13 is a diagram that represents a comparison between the number of errors obtained from the administration of the kit of the invention to a number of male controls, and the number of errors obtained from the administration of the same kit to a group of male patients suffering from Parkinson's disease, in the 50 to 59 age range;
- Figure 14 is a diagram that represents a comparison between the number of errors obtained from the administration of the kit of the invention to a number of male controls, and the number of errors obtained from the administration of the same kit to a group of male patients suffering from Parkinson's disease, in the 60 to 69 age range;
- Figure 15 is a diagram that represents a comparison between the number of errors obtained from the administration of the kit of the invention to a number of male controls, and the number of errors obtained from the administration of the same kit to a group of male patients suffering from Parkinson's disease, in the 70 to 79 age range;
- Figure 16 is a diagram that represents the consistency of the results obtained when the kit of the invention is administered by a doctor (test) with the results obtained when the same kit is administered to the same subjects by paramedical personnel (retest);
- Figure 17 is a diagram that represents the consistency of the results obtained when the kit of the invention is self-administered by the subject under examination (test) with the results obtained when the same kit is administered to the same subjects by a doctor (retest).
   Thus, in a first aspect, the present invention relates to a kit comprising a set of 25 to 45 testers and directions for use of the kit, wherein
- each tester comprises a single odorant, other than those of the other testers, adhered to an appropriate odorless substrate common to all testers,
- each tester carries a tester identification mark,
- all testers have the same format, colors, size and texture feel, the only distinctive aspect thereamong being said identification mark, which uniquely corresponds to the single odorant adhered to each tester,
- the area delimited by the odorant adhered on each tester, as well as its color and position on the substrate are common to all testers.
   Preferably, said 25 to 45 odorants for each tester are selected from the group comprising cloves; rose; lavender; banana; pine; mushroom; talc; mint; coconut; strawberry candy; apple; cheese; watermelon; grass; lilac; sage; licorice; washing soap; wood-like aroma; coffee-coffee liqueur; chocolate-cocoa; oregano; basil; rosemary; garlic; lemon; peach; olibanum; orange; anise-Sambuca; pineapple-pineapple juice; balsamic eucalyptol pastille; unpleasant odor like that of decomposing organic matter; peanuts; butter; apricot; chamomile; smoked charcuterie-speck; onion; car/bike tire rubber; carrot; pear; tangerine; parsley; and bitter almonds.
   More preferably, at least 12 of said 25 to 45 odorants are selected from the group comprising bitter almonds; apple; apricot; mushroom; car/bike tire rubber; butter; lilac; sage; onion; smoked charcuterie-speck; coffee-coffee liqueur; basil; chamomile; rosemary; balsamic eucalyptol pastille; talc; olibanum; unpleasant odor like that of decomposing organic matter; and lavender.
   In one preferred embodiment, the odorant is in microencapsulated form, which affords convenient storage, without any loss or degradation prior to use, as shown in the following examples. Nonetheless, further advantageous forms may be provided, in which the aroma is maintained unchanged prior to use for convenient storage. For example, the odorant may be introduced in liquid form into small, disposable flexible sealed packets made of plastic, aluminum or the like. Thus, the appropriately marked packet will be used as a tester for the purposes of the present invention. The packet may be opened, for instance, by manual tearing. Therefore, the odorant is smelled through the opening formed in the packet, while remaining therein. Otherwise, the odorant may be placed in liquid form, in combination with a small odorless plastic ball within small plastic blisters flat on one side. Such plastic blisters are closed on one side with aluminum material. The odorant may be smelled by simply pressing the convex plastic side of the blister (like for drug tablets pressed out of their blisters). This allows the odorless plastic ball in the bluster to break the aluminum closure and release the few µl of odorant required. Each appropriately marked plastic blister may be used as a tester for the purposes of the present invention.
   The substrate against which the odorant is adhered is an appropriate odorless material, because no interference should be allowed with the perception of the specific odor. Preferably, the substrate against which the odorant is adhered is an odorless card or a plate of odorless plastic material. In a preferred embodiment, said substrate is a square or rectangular card, having one surface with the odorant adhered thereto, and the other surface with nothing on it.
   For discrimination of the testers in the kit of the invention, each tester carries a mark other than those of the other testers. Particularly, and preferably, said mark is a number, a letter, a symbol or a combination thereof. More preferably, the mark is an integer.
   The kit of the invention also includes directions of use comprising
- information about the use of said kit, and
- a list of items.

As used herein, the term "item" is intended to designate a system composed of a set of at least three possible answers to the question "This smell is or resembles more to?" or other similar questions. Each set of said at least three answers has the same mark as the tester with which the item is associated. Therefore, the total number of items is equal to the total number of testers of one kit. Also, only one of said at least three possible answers is right, i.e. only one corresponds to the odorant adhered to the tester with which the item is associated by a common mark. Preferably, each item consists of a set of four possible answers.

In one preferred embodiment, each tester and its item are designated by an integer.

Preferably, the kit of the invention includes a set of 30 to 40 testers, such range having been found, as shown in the examples below, a convenient and advantageous compromise between the reliability of olfactory response results and the level of attention that the subjects are required to have during the test. More preferably, the kit of the invention comprises a set of 33 testers, as the best compromise between the two above requirements.

In one preferred embodiment, said list is composed of the following items, each marked by an integer, with a single tester corresponding thereto, and marked by the same integer:

| | | | | | | |
|---|---|---|---|---|---|---|
| 1 | a. | LICORICE | | 18 | a. | WASHING SOAP |
| | b. | CLOVES | | | b. | CHOCOLATE |
| | c. | ONION | | | c. | SMOKED CHARCUTERIE |
| | d. | BANANA | | | d. | ROSEMARY |
| | | | | | | |
| 2 | a. | BASIL | | 19 | a. | PICKLES |
| | b. | ROSE | | | b. | WOOD-LIKE AROMA |
| | c. | GASOLINE | | | c. | SMOKED CHARCUTERIE |
| | d. | ORANGE | | | d. | TEA |
| | | | | | | |
| 3 | a. | CHAMOMILE | | 20 | a. | TOBACCO |
| | b. | COFFEE | | | b. | TEA |
| | c. | SMOKED CHARCUTERIE - SPECK | | | c. | UNPLEASANT, SOLPHUROUS ROTTEN EGG ODOR |
| | d. | LAVENDER | | | d. | COFFEE-COFFEE LIQUEUR |
| | | | | | | |
| 4 | a. | CHEESE | | 21 | a. | TEA |
| | b. | GASOLINE | | | b. | PUTRID, SULPHUROUS ODOR |
| | c. | BANANA | | | c. | CHOCOLATE - COCOA |
| | d. | BUTTER | | | d. | CHAMOMILE |
| | | | | | | |
| 5 | a. | PINE | | 22 | a. | GARLIC |
| | b. | COFFEE | | | b. | ORANGE |
| | c. | MIMOSA FLOWERS | | | c. | OREGANO |
| | d. | ORANGE | | | d. | VANILLA |
| | | | | | | |
| 6 | a. | CIGARETTE SMOKE | | 23 | a. | ORANGE |
| | b. | CHAMOMILE | | | b. | BASIL |
| | c. | FRUIT CANDIES | | | c. | ONION |
| | d. | MUSHROOM | | | d. | VANILLA |
| | | | | | | |
| 7 | a. | MINT | | 24 | a. | ROSEMARY |
| | b. | COFFEE | | | b. | SMOKED CHARCUTERIE - SPECK |
| | c. | TALC | | | c. | BREAD |
| | d. | FRUIT CANDY | | | d. | PEACH |
| | | | | | | |
| 8 | a. | PICKLES | | 25 | a. | MINT |
| | b. | MINT | | | b. | GASOLINE |
| | c. | COFFEE | | | c. | TOBACCO - CIGARETTE |
| | d. | WATERMELON | | | d. | GARLIC |
| | | | | | | |
| 9 | a. | SAGE | | 26 | a. | MOLD |
| | b. | COCONUT | | | b. | LEMON |
| | c. | GASOLINE | | | c. | VANILLA |
| | d. | WATERMELON | | | d. | PICKLES |
| 10 | a. | PEANUTS | | 27 | a. | PEACH |
| | b. | STRAWBERRY CANDY | | | b. | MOLD |
| | c. | GASOLINE | | | c. | TOBACCO - CIGARETTE |
| | d. | SMOKED CHARCUTERIE | | | d. | FUEL OIL |
| | | | | | | |
| 11 | a. | ORANGE | | 28 | a. | WATERMELON |
| | b. | CHOCOLATE | | | b. | SMOKED CHARCUTERIE - SPECK |
| | c. | PUTRID, SULPHUROUS | | | c. | OLIBANUM |
| | | ODOR | | | d. | ORANGE |
| | d. | APPLE - APPLE SHAMPOO | | | | |
| | | | | | | |
| 12 | a. | ONION | | 29 | a. | KITCHEN GAS |
| | b. | FUEL OIL | | | b. | ORANGE - ORANGE CANDY |
| | c. | CHEESE AROMA | | | c. | MOLD |
| | d. | ACETONE | | | d. | GASOLINE |
| | | | | | | |
| 13 | a. | SMOKED CHARCUTERIE | | 30 | a. | PINEAPPLE |
| | b. | WATERMELON | | | b. | CHAMOMILE |
| | c. | CHOCOLATE | | | c. | ANISE - SAMBUCA |
| | d. | BASIL | | | d. | PUTRID, SULPHUROUS ODOR |
| | | | | | | |
| 14 | a. | GRASS | | 31 | a. | CIGARETTE SMOKE |
| | b. | VANILLA | | | b. | KITCHEN GAS |
| | c. | ROSEMARY | | | c. | PINEAPPLE - PINEAPPLE JUICE |
| | d. | GASOLINE | | | d. | CHAMOMILE |
| | | | | | | |
| 15 | a. | CHOCOLATE | | 32 | a. | BALSAMIC PASTILLE |
| | b. | LILAC AROMA | | | b. | TOBACCO - CIGARETTE |
| | c. | SMOKED CHARCUTERIE | | | c. | PICKLES |
| | d. | COFFEE | | | d. | PUTRID, SULPHUROUS ODOR |
| | | | | | | |
| 16 | a. | SAGE | | 33 | a. | VANILLA - CREAM |
| | b. | CHAMOMILE | | | b. | APRICOT |
| | c. | SMOKED CHARCUTERIE | | | c. | PEAR |
| | d. | GARLIC | | | d. | UNPLEASANT ODOR - like that of decomposing organic matter |
| | | | | | | |
| 17 | a. | PUTRID ODOR - ROTTEN EGGS | | | | |
| | b. | LICORICE | | | | |
| | c. | KITCHEN GAS | | | | |
| | d. | COFFEE | | | | |

Preferably, the information provided in the directions for use of the kit are as follows:
(a) Take the tester marked as the first one among those in the kit;
(b) read the answers provided in the item with the same mark as the tester;
(c) using a piece of paper, rub the odorant on the tester;
(d) bring the rubbed odorant on the tester to your nose, about 1 cm from your nostrils, and smell while avoiding any contact between the tester and the nostrils; while sniffing, read again and think about the answers provided for the item;
(e) select one answer (the answer that is believed to be the right one) to the question: "This smell is or resembles more to?";
(f) without waiting too much between one item and the other, but not too quickly, take the tester marked as the second one among those in the kit;
(g) repeat the steps from (b) to (e) for the tester and item marked as the second ones and then for each tester in the kit, following the order of the marks on the testers and items and always using a new piece of paper to rub each tester.

As confirmed by the following examples, such information conveniently and advantageously allows collection of olfactory response data even without requiring the presence of specially skilled personnel, i.e. doctors or paramedical personnel, while ensuring high response reliability and test repeatability.

Furthermore, the indication under (c) of rubbing the microencapsulated odorant by a piece of paper conveniently allows the microcapsules to be broken and release the aroma by a light rubbing action. More invasive means, such as rigid pointed, flat-pointed or strongly abrasive objects are not suitable for the purpose, because they are liable to damage the tester, even without sufficiently breaking the microcapsules, like in the case of pointed objects.

Should the tester inadvertently contact the nose skin, it is recommended to rub the nostril skin with a water moistened handkerchief or tissue. After that, the test may be continued and terminated.

Preferably, the directions for use of the kit may be in the form of written text, images, pictures, videos or a combination thereof. Furthermore, such directions for use may be conveniently provided in paper form or on optical media, such as CD-ROM or DVD, or through remote consultation, such as from an appropriate Internet or Intranet site, or a combination thereof. In this case, the step described under (e) of the above directions for use, which requires the selection of one answer (i.e. the answer that is believed to be the right one) to the question: "This smell is or resembles more to?" may be carried out by striking through, circling, filling in or otherwise marking the selected answer, appropriately depending on the media on which such directions are provided, and in any case in a perfectly clear and unmistakable manner.

In another aspect, the present invention relates to the use of testers for the preparation of a kit of the invention for collecting olfactory response data to be employed in the diagnosis of neurological diseases.

In another aspect, the present invention relates to a method for collection and assessment of olfactory responses in the diagnosis of neurological diseases, said method including the steps of:
a) administering a kit of the invention;
b) collecting the list of answers for each item, when all answers have been given in the same order as provided in the list, according to the directions for use of the kit;
c) comparing the collected answers with the right answers;
d) counting the number of wrong answers in the list of answers and comparing such number with the test reference values, expressed as a maximum number of errors.

A few embodiments of the inventive kit and of the assessment of the olfactory responses obtained thereby will be now provided by way of example and without limitation.

Example 1: Preparing tester sets; Using tester sets; Checking the intensity of olfactory stimulation

### Preparing tester sets

A panel of 45 odors was selected, in view of the cultural background of the Italian population (Table 2).

| Table 2. Selected odors | |
|---|---|
| 1. | CLOVES |
| 2. | ROSE |
| 3. | LAVENDER |
| 4. | BANANA |
| 5. | PINE |
| 6. | MUSHROOM |
| 7. | TALC |
| 8. | MINT |
| 9. | COCONUT |
| 10. | STRAWBERRY CANDY |
| 11. | APPLE |
| 12. | CHEESE |
| 13. | WATERMELON |
| 14. | GRASS |
| 15. | LILAC |
| 16. | SAGE |
| 17. | LICORICE |
| 18. | WASHING SOAP |
| 19. | WOOD-LIKE AROMA |
| 20. | COFFEE-COFFEE LIQUEUR |
| 21. | CHOCOLATE - COCOA |
| 22. | OREGANO |
| 23. | BASIL |
| 24. | ROSEMARY |
| 25. | GARLIC |
| 26. | LEMON |
| 27. | PEACH |
| 28. | OLIBANUM |
| 29. | ORANGE |
| 30. | ANISE - SAMBUCA |
| 31. | PINEAPPLE - PINEAPPLE JUICE |
| 32. | BALSAMIC EUCALYPTOL PASTILLE |
| 33. | UNPLEASANT ODOR (like that of decomposing organic matter) |
| 34. | PEANUTS |
| 35. | BUTTER |
| 36. | APRICOT |
| 37. | CHAMOMILE |
| 38. | SMOKED CHARCUTERIE - SPECK |
| 39. | ONION |
| 40. | Car/bike TIRE - RUBBER |
| 41. | CARROT |
| 42. | PEAR |
| 43. | TANGERINE |
| 44. | PARSLEY |
| 45. | BITTER ALMONDS |

The odorants were in microencapsulated form according to prior art methods and were applied to suitable substrates, particularly, in this Example, in a card, to form the testers of the present invention.

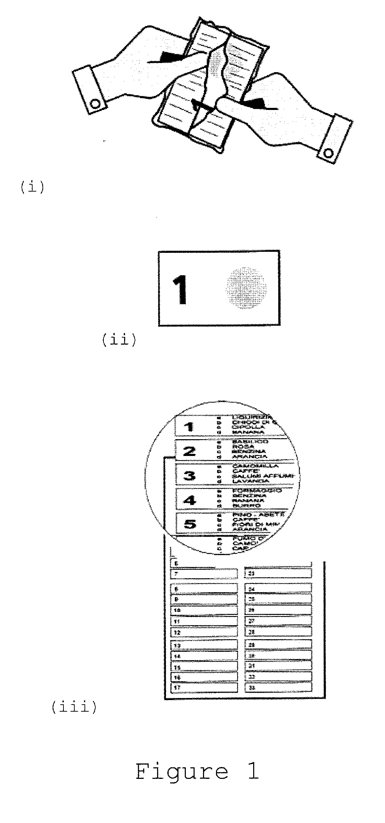

Each tester was provided in either one of the two following formats:
Q) The microencapsulated odorant was adhered to the ivory-white surface of a 2 x 2 cm square cardboard substrate. The other surface of the square card was totally odorless. 45 testers were prepared for each test, each having one of the odors of Table 2, different from the others. The area delimited by the adhered microencapsulated odorant and its position on the substrate surface were common to all 45 testers, in terms of both size and shape (e.g. square, round, oval and the like). Each tester was introduced in a packet and closed, the packet bearing the number corresponding to the odor, according to Table 2. Thus, for example, the packet no. 1 contained the tester of the odor no. 1, i.e. cloves.
R) The microencapsulated odorant was adhered to a 6.5 x 3.5 surface of a rectangular substrate (see Figure 1 (i) and (ii)), the substrate surface itself bearing the number corresponding to that odor according to Table 2. The other surface of the tester substrate had no odor or writing thereon. 45 testers were prepared for each test, each having one of the odors of Table 2, different from the others. The area delimited by the adhered microencapsulated odorant and its location on the substrate surface were common to all 45 testers, in terms of both position, size and shape (e.g. square, round, oval and the like).

The testers had the same color, i.e. the color of the card, the same color and format of the number written thereon, the same color, shape, position and size of the area on which the microencapsulated aroma is located. The different odorants have the same neutral color (e.g. ivory-white). The testers are only differentiated by the number that designates them and by the odor that corresponds to that identification number (Table 2). Each tester is physical separated from the others and may be inserted in a packet.

Then, 1000 sets of 45 Q-format testers and 300 sets of 45 R-format testers were prepared.

### Using tester sets

The microencapsulated odorant on the tester was smelled as follows: 1) the area with the microencapsulated odorant was rubbed lightly 3-4 times using a small piece of paper, to break the microcapsules and release the aroma; 2) then, the rubbed area was brought to 1 cm from the nostrils, without touching the nostril skin with the tester and 3) the area was sniffed one or more times. If needed, the procedure was started anew. Then, the spent testers and paper pieces were thrown into a bin with a cover, about one meter and a half on one side of the table used for the experiment.

A new piece of paper had to be used for each tester, to rub the microencapsulated odorant thereon, to prevent any olfactory contamination with the preceding odorant.

Also, the tester sniffing sequence had to match the sequence provided in Table 2. Therefore, the first tester to be used was the one marked as number 1, the second was the one marked as number 2 and so on to the tester number 45.

### Checking the intensity of olfactory stimulation

Thirty-two healthy adults (16 men and 16 women. Average age of men 55.81, SD ± 8,87. Average age of women 56.13, SD ± 10.22), not suffering from either acute or chronic diseases of any organ or apparatus, and being found negative, upon otorhinolaringological examination, to any rhinosinusopathia, were submitted to a check of the intensity of the olfactory stimulation released by the testers. Each subject was taught the procedure of use of the 45 testers and his/her understanding of the method was verified. Then, each subject self-administered the set of 45 testers (odors in Table 2). Ten men and ten women received a set of 45 Q-format testers each, and other six men and six women received a set of 45 R-format testers.

The individuals under examination were required to state, for each of the 45 testers, whether the sniffed odor could be defined:
A) distinctly perceivable;
B) too weak and faintly perceivable.

All the subjects under examination gave the answer A.

Thus, each tester prepared as described above and correctly employed according to the directions for use, was found to be able to release the aroma with an intensity well above the threshold and hence easily perceivable by the human olfactory system.

Example 2: Preparing kits of the invention; preparing a multiple and forced choice matrix

### Preparing kits of the invention

This Example was based on the use of the testers no. 1 to no. 33 as described above, which correspond to the odors no. 1 to no. 33 listed in Table 2 and listed again in the same order in Table 3 for convenience.

Namely, 900 sets of 33 R-format testers, carrying odorants from no. 1 to no. 33 of Table 3, were prepared.

| Table 3 | |
|---|---|
| No. of the odor and no. on the tester | Corresponding odor |
| 1. | CLOVES |
| 2. | ROSE |
| 3. | LAVENDER |
| 4. | BANANA |
| 5. | PINE |
| 6. | MUSHROOM |
| 7. | TALC |
| 8. | MINT |
| 9. | COCONUT |
| 10. | STRAWBERRY CANDY |
| 11. | APPLE |
| 12. | CHEESE |
| 13. | WATERMELON |
| 14. | GRASS |
| 15. | LILAC |
| 16. | SAGE |
| 17. | LICORICE |
| 18. | WASHING SOAP |
| 19. | WOOD-LIKE AROMA |
| 20. | COFFEE-COFFEE LIQUEUR |
| 21. | CHOCOLATE - COCOA |
| 22. | OREGANO |
| 23. | BASIL |
| 24. | ROSEMARY |
| 25. | GARLIC |
| 26. | LEMON |
| 27. | PEACH |
| 28. | OLIBANUM |
| 29. | ORANGE |
| 30. | ANISE - SAMBUCA |
| 31. | PINEAPPLE - PINEAPPLE JUICE |
| 32. | balsamic eucalyptol pastille |
| 33. | UNPLEASANT ODOR - decomposing organic matter |

### Preparing a multiple, forced choice matrix

A matrix of 33 items, one for each of the 33 testers, was written on a piece of paper, physically separated from the testers (which are in turn separated from each other). For each tester, 4 answers (or descriptors) were provided, one right answer and three wrong answers. The right answer was the name of the odor on that tester. The three wrong answers were odors that also belonged to the Italian cultural background, but were definitely different from the odor on the tester.

Therefore, the kit of the present Example comprised a set of 33 testers and a list of 33 items, each composed of four answer options, one right answer and three wrong answers, each corresponding to a letter of the alphabet: a., b., c., d., listed on a sheet separate from the tester (see Table 4 below); each item was associated with the following question: "This smell is or resembles more to?" (Table 4).

### Procedure of use

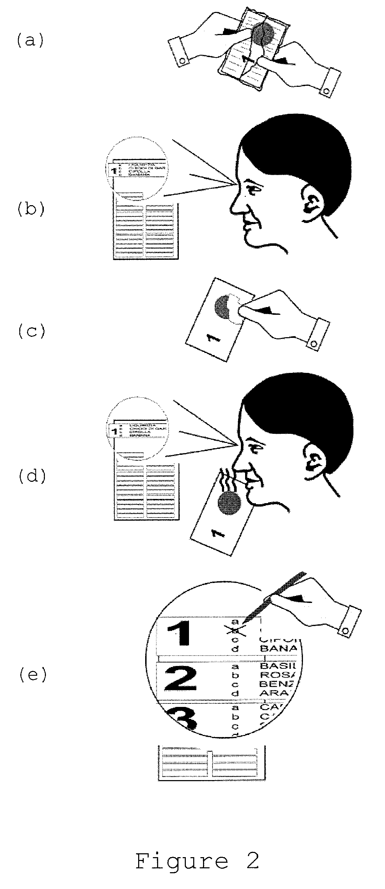

The directions for proper use of the kit included the steps of:
- starting from item no. 1 and continuing in ascending order to item no. 33;
- removing the tester from its packaging enclosure;
- reading the answers suggested for the item being examined;
- making sure of actually reading the answers suggested for the tester to be used (e.g.: item no. 1 answers for the tester no. 1);
- releasing the odor from the tester, by rubbing the area with the microencapsulated odorant with a piece of paper, as described in Example 1;
- bringing the odorant area of the tester to about 1 cm from the nostrils, while avoiding any contact with the nose skin, and sniffing one or more times. While sniffing, reading the four answers suggested for that tester, and hence for that item;
- before giving the response for that item, the procedure can be repeated. For this purpose, the answer has to be selected by ticking or circling the answer a, b, c, or d (see Figure 2). Once the answer has been selected, there will be no longer the possibility to correct neither the answer given for that tester, and the corresponding item, nor for those that have already been administered/self-administered for which an answer has been already given;
- throwing the spent tester and the paper piece used for rubbing that tester into a container (bin with a cover), about one meter and a half from the subject being examined.

An answer must always be given, even when no smell has been perceived or recognized. In this case, a choice must be made among the suggested options.

The test has no time restrictions, wherefore the subject can think and sniff as long as he/she wishes before giving an answer, for each item. Nonetheless, there should be no pause between the selection of an answer and the administration of the next tester. The test shall always be administered from the tester no. 1 to the last tester, i.e. the tester no. 33 in this

### Example.

A new piece of paper has to be used for each tester, to rub the microencapsulated odorant thereon, to prevent any olfactory contamination with the preceding odorant. Some aroma remains on the piece of paper that is used to rub the tester.

The following Table 4 lists the multiple-choice matrix. The number that designates each group of four answers (a, b, c, d) identifies the corresponding tester with which it is uniquely associated. Also, the right odor for each tester is named and numbered in Table 3. The tester no. 1 corresponds to the odor no. 1 listed in Table 3 and so on.

**Table 4**

| | | | | | | |
|---|---|---|---|---|---|---|
| 1 | a. | LICORICE | | 18 | a. | WASHING SOAP |
| | b. | CLOVES | | | b. | CHOCOLATE |
| | c. | ONION | | | c. | SMOKED CHARCUTERIE |
| | d. | BANANA | | | d. | ROSEMARY |
| | | | | | | |
| 2 | a. | BASIL | | 19 | a. | PICKLES |
| | b. | ROSE | | | b. | WOOD-LIKE AROMA |
| | c. | GASOLINE | | | c. | SMOKED CHARCUTERIE |
| | d. | ORANGE | | | d. | TEA |
| | | | | | | |
| 3 | a. | CHAMOMILE | | 20 | a. | TOBACCO |
| | b. | COFFEE | | | b. | TEA |
| | c. | SMOKED CHARCUTERIE - SPECK | | | c. | UNPLEASANT, SOLPHUROUS ROTTEN EGG ODOR |
| | d. | LAVENDER | | | d. | COFFEE-COFFEE LIQUEUR |
| | | | | | | |
| 4 | a. | CHEESE | | 21 | a. | TEA |
| | b. | GASOLINE | | | b. | PUTRID, SULPHUROUS ODOR |
| | c. | BANANA | | | c. | CHOCOLATE - COCOA |
| | d. | BUTTER | | | d. | CHAMOMILE |
| | | | | | | |
| 5 | a. | PINE | | 22 | a. | GARLIC |
| | b. | COFFEE | | | b. | ORANGE |
| | c. | MIMOSA FLOWERS | | | c. | OREGANO |
| | d. | ORANGE | | | d. | VANILLA |
| | | | | | | |
| 6 | a. | CIGARETTE SMOKE | | 23 | a. | ORANGE |
| | b. | CHAMOMILE | | | b. | BASIL |
| | c. | FRUIT CANDY | | | c. | ONION |
| | d. | MUSHROOM | | | d. | VANILLA |
| | | | | | | |
| 7 | a. | MINT | | 24 | a. | ROSEMARY |
| | b. | COFFEE | | | b. | SMOKED CHARCUTERIE - SPECK |
| | c. | TALC | | | c. | BREAD |
| | d. | FRUIT CANDY | | | d. | PEACH |
| | | | | | | |
| 8 | a. | PICKLES | | 25 | a. | MINT |
| | b. | MINT | | | b. | GASOLINE |
| | c. | COFFEE | | | c. | TOBACCO - CIGARETTE |
| | d. | WATERMELON | | | d. | GARLIC |
| | | | | | | |
| 9 | a. | SAGE | | 26 | a. | MOLD |
| | b. | COCONUT | | | b. | LEMON |
| | c. | GASOLINE | | | c. | VANILLA |
| | d. | WATERMELON | | | d. | PICKLES |
| | | | | | | |
| 10 | a. | PEANUTS | | 27 | a. | PEACH |
| | b. | STRAWBERRY CANDY | | | b. | MOLD |
| | c. | GASOLINE | | | c. | TOBACCO - CIGARETTE |
| | d. | SMOKED CHARCUTERIE | | | d. | FUEL OIL |
| | | | | | | |
| 11 | a. | ORANGE | | 28 | a. | WATERMELON |
| | b. | CHOCOLATE | | | b. | SMOKED CHARCUTERIE - SPECK |
| | c. | PUTRID, SULPHUROUS ODOR | | | c. | OLIBANUM |
| | d. | APPLE - APPLE SHAMPOO | | | d. | ORANGE |
| | | | | | | |
| 12 | a. | ONION | | 29 | a. | KITCHEN GAS |
| | b. | FUEL OIL | | | b. | ORANGE - ORANGE CANDY |
| | c. | CHEESE AROMA | | | c. | MOLD |
| | d. | ACETONE | | | d. | GASOLINE |
| | | | | | | |
| 13 | a. | SMOKED CHARCUTERIE | | 30 | a. | PINEAPPLE |
| | b. | WATERMELON | | | b. | CHAMOMILE |
| | c. | CHOCOLATE | | | c. | ANISE - SAMBUCA |
| | d. | BASIL | | | d. | PUTRID, SULPHUROUS ODOR |
| | | | | | | |
| 14 | a. | GRASS | | 31 | a. | CIGARETTE SMOKE |
| | b. | VANILLA | | | b. | KITCHEN GAS |
| | c. | ROSEMARY | | | c. | PINEAPPLE - PINEAPPLE JUICE |
| | d. | GASOLINE | | | d. | CHAMOMILE |
| | | | | | | |
| 15 | a. | CHOCOLATE | | 32 | a. | BALSAMIC PASTILLE |
| | b. | LILAC AROMA | | | b. | TOBACCO - CIGARETTE |
| | c. | SMOKED CHARCUTERIE | | | c. | PICKLES |
| | d. | COFFEE | | | d. | PUTRID, SULPHUROUS ODOR |
| | | | | | | |
| 16 | a. | SAGE | | 33 | a. | VANILLA - CREAM |
| | b. | CHAMOMILE | | | b. | APRICOT |
| | c. | SMOKED CHARCUTERIE | | | c. | PEAR |
| | d. | GARLIC | | | d. | UNPLEASANT ODOR - like that of decomposing organic matter |
| | | | | | | |
| 17 | a. | PUTRID ODOR - ROTTEN EGGS | | | | |
| | b. | LICORICE | | | | |
| | c. | KITCHEN GAS | | | | |
| | d. | COFFEE | | | | |

Example 3: Administering the test to a group of healthy subjects (controls) of different age and sex classes.

In the present Example, the test was administered by a neurologist and an otolaryngologist. The latter had conducted a well-targeted anamnestic study and subjected to anterior rhinoscopy all the subjects selected for the test, to exclude any rhinosinusopathia. The examined subjects did not suffer from acute or chronic diseases of any organ or apparatus, showed no sign of mental decay and all had a score over 27 on the Mini Mental State Examination (#30). In these healthy subjects, referred to as "Controls", the number of errors was examined in 5 ten-year age ranges (30-39; 40-49; 50-59; 60-69; 70-79) separately for men and women. The number of healthy subjects being examined, divided by age classes and sex, is indicated in the tables below. The differences among age classes in the number of errors made during the test was evaluated using the non-parametric Kruskal-Wallis test and the analysis of variance (ANOVA). Multiple comparisons were made by the Tukey test. The ratio of the number of errors to age was also assessed by a smoothing spline (statistical method).

### Results:

### Number of errors in controls by sex and age class

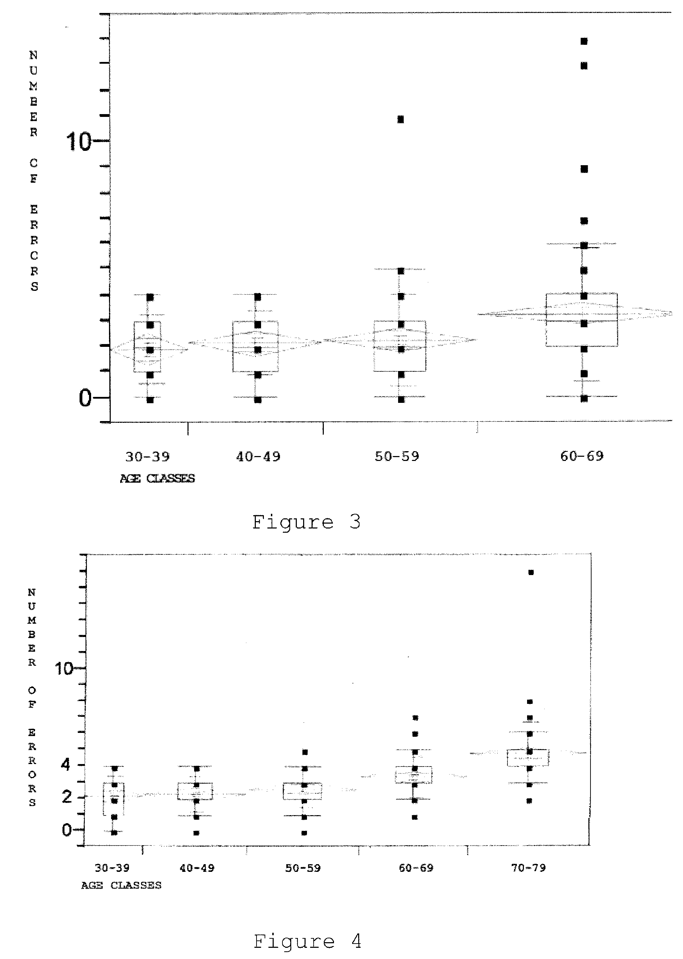

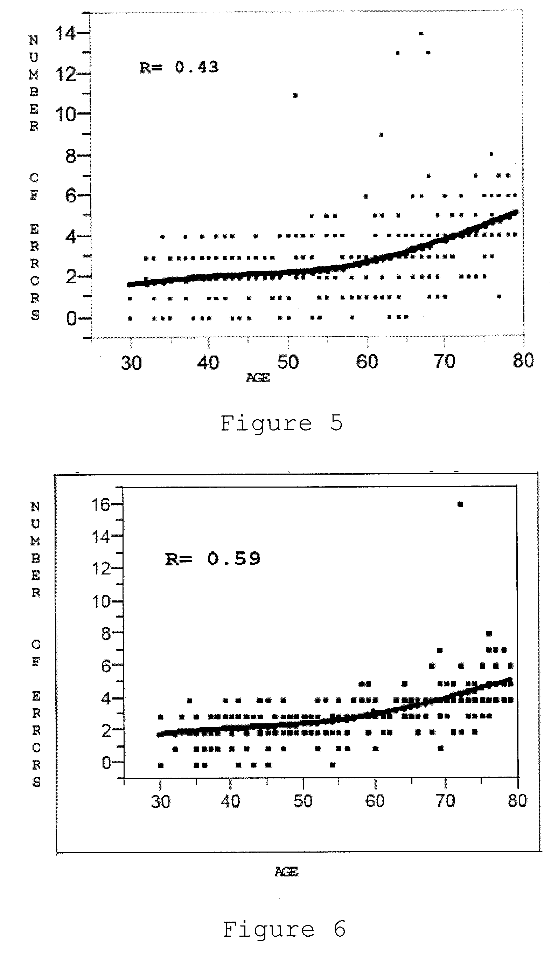

No significant difference was found in the number of errors between sexes. The number of errors significantly increased (p<0.0001) with age in both female and male subjects (Tables 5 and 6, Figures 3 and 4). Particularly, in both sexes the number of errors in the 70-79 age class was found to be significantly higher than that of the 60-69 age class (p<0.01) and those of the three classes below 60 (p<0.01), whereas the number of errors in test response recorded for the 60-69 age class was found in turn to be significantly higher than that of the three classes below 60 (p<0.05). No significant difference was found between the 50-59, 40-49, 30-39 age classes. The age/number of errors ratio is shown in Figures 5 and 6 separately for both sexes. The two curves (*Smoothing Spline*) show that the increase in the number of errors is higher after the age of 60.

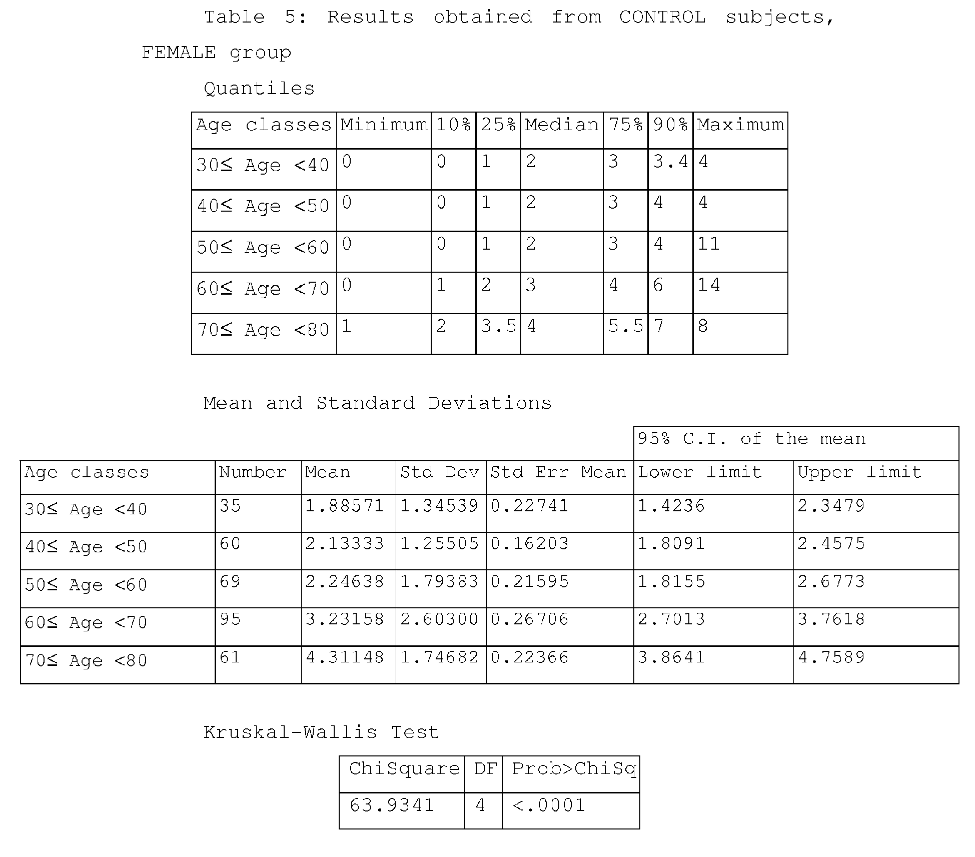

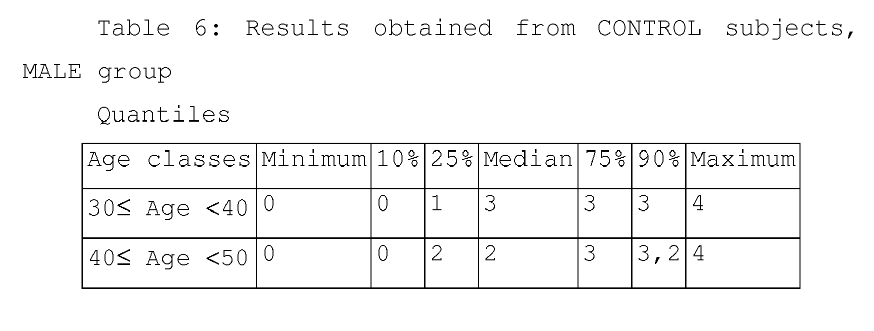

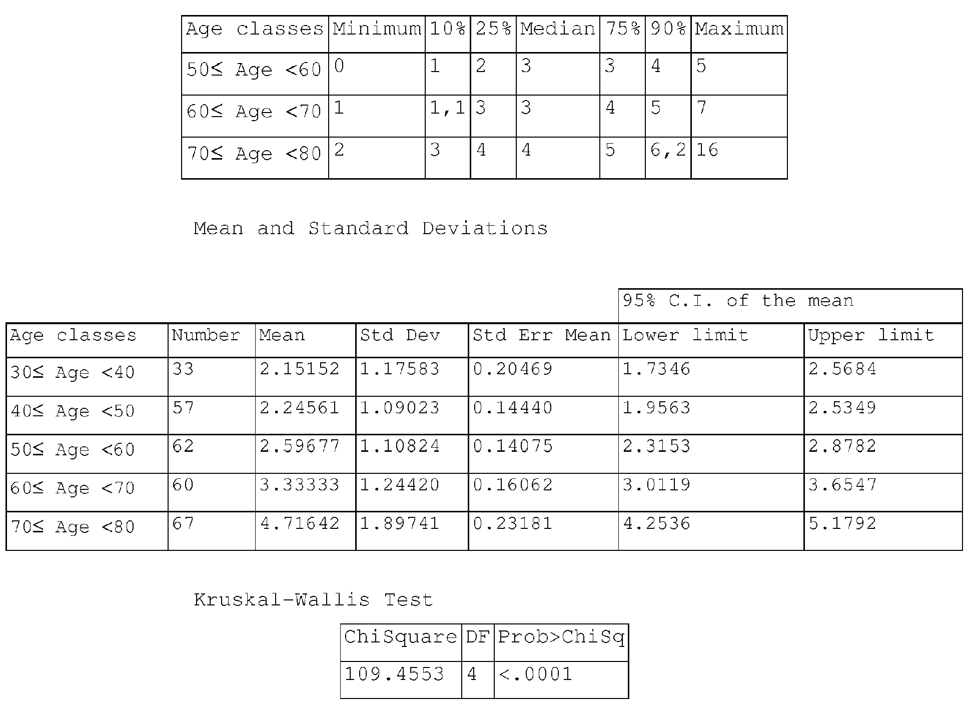

Example 4: Administering the kit of the invention to healthy subjects (controls) and patients suffering from Parkinson's disease

Kits prepared according to Example 2, comprising sets of 33 testers, were used.

The subjects under examination (both healthy subjects and patients) belonged to three ten-year age classes: 50-59, 60-69, 70-79. In each age class, the controls (i.e. the healthy subjects) and the Parkinson's disease patients were 131 and 25, 155 and 47, 128 and 61 respectively. The healthy subjects and the patients were evaluated by a neurologist and an otolaryngologist who had conducted a well-targeted anamnestic study and subjected all the subjects (healthy subjects and patients) to anterior rhinoscopy, to exclude individuals suffering from rhinosinusopathia. The controls and patients had a score over 27 on the Mini Mental State Examination (see reference #30). The subjects with Parkinson's disease were treated in neurological centers dedicated to the study and treatment of this disease. The criteria suggested by Gelb et al. were used for the diagnosis of Parkinson's disease (see reference #80).

The differences in numbers of errors in test response between controls and Parkinson's disease patients were assessed both by age class and by a combination of sex and age class, using the non-parametric Wilcoxon test for independent data.

### Results:

### Differences between Parkinson patients and Controls

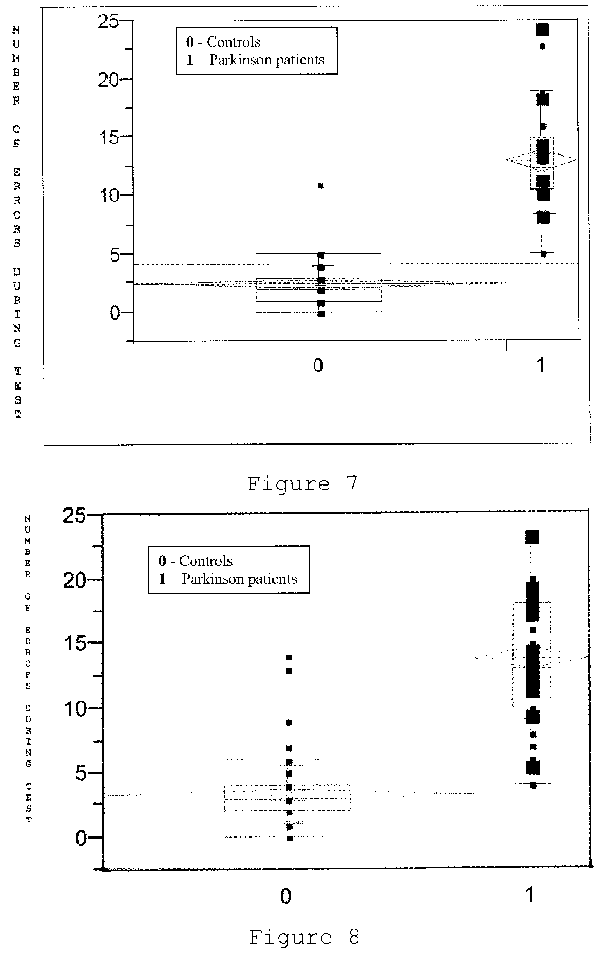

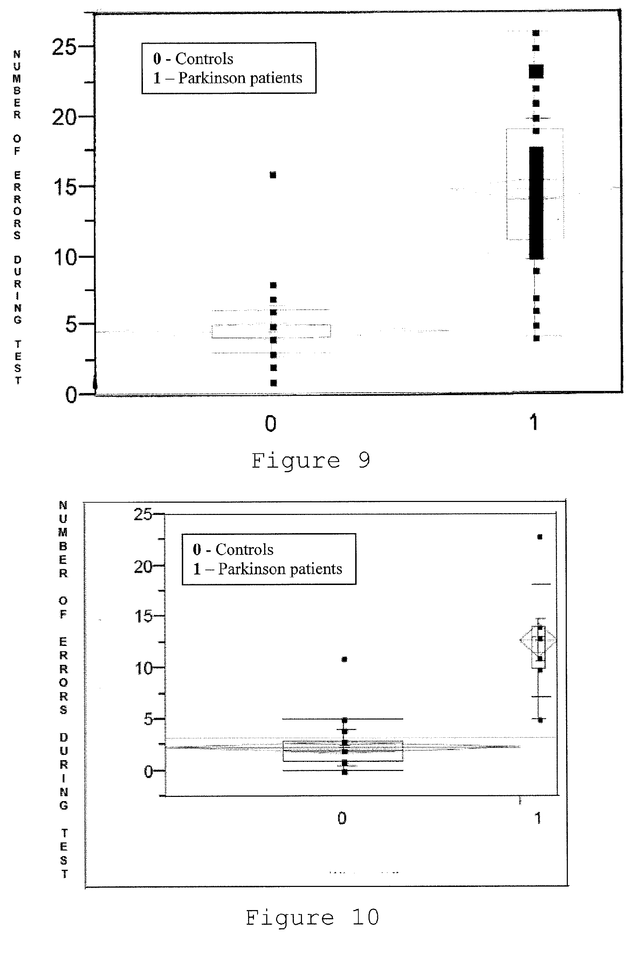

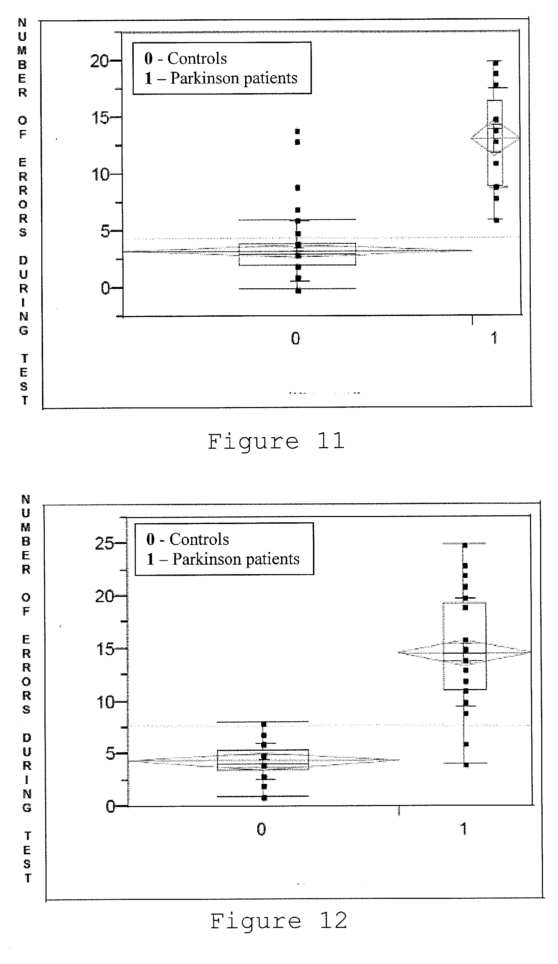

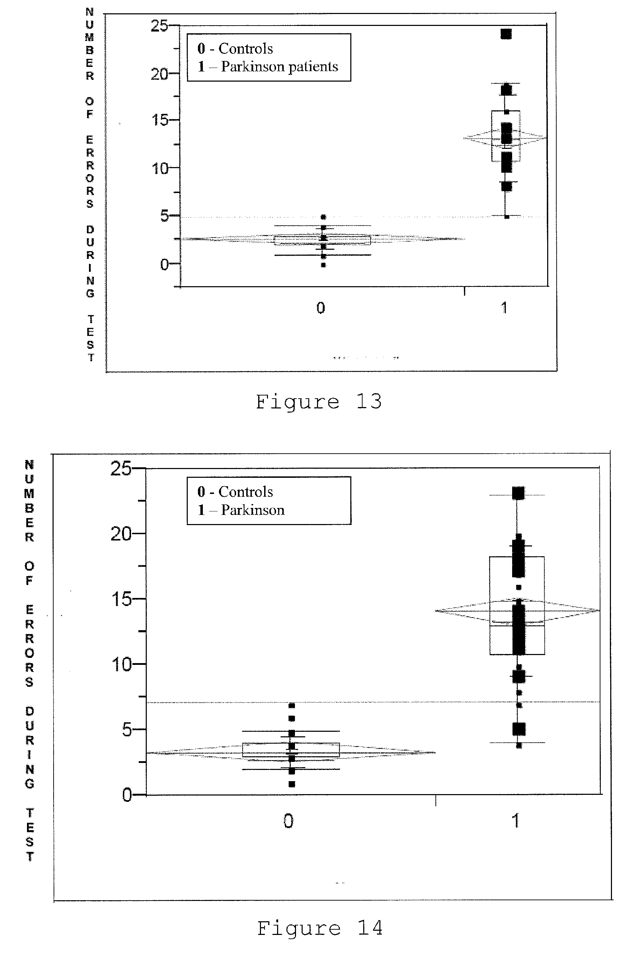

In all three age classes under examination, the Parkinson's disease patients showed a number of errors significantly higher than Controls (p<0.0001)(Tables 7-9, Figures 7-9). This difference was found both in female and in male subjects (Tables 10-15, Figures 10-15).

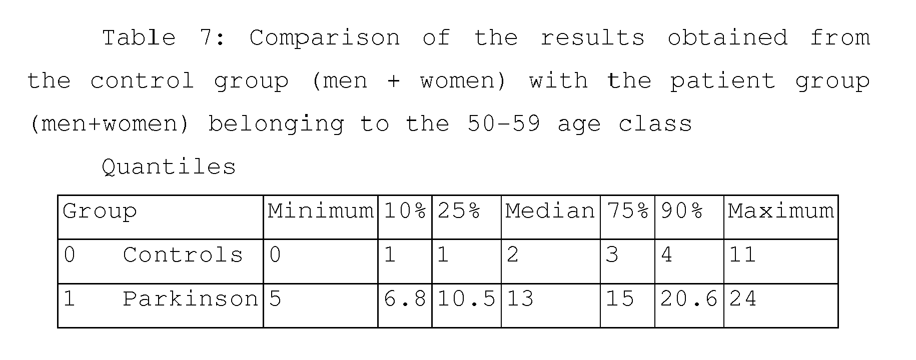

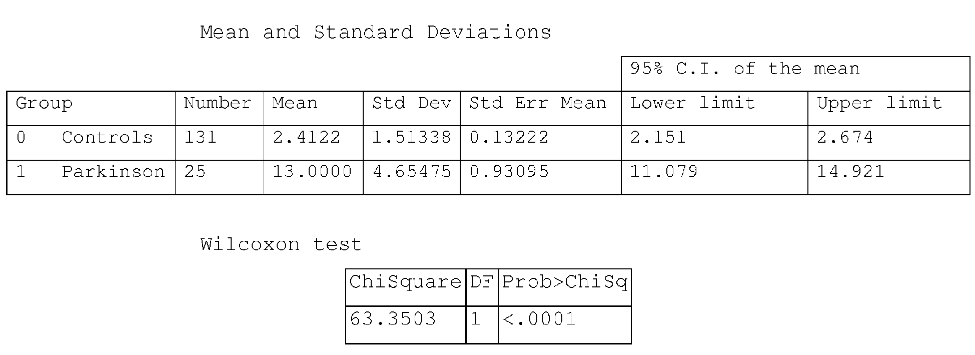

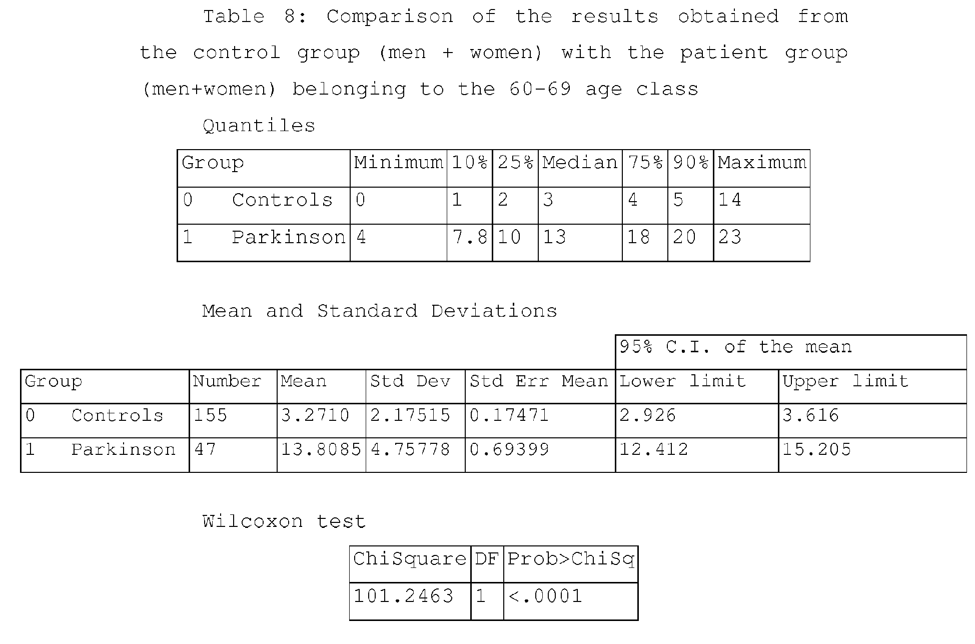

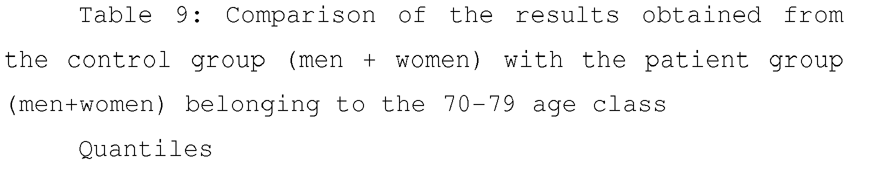

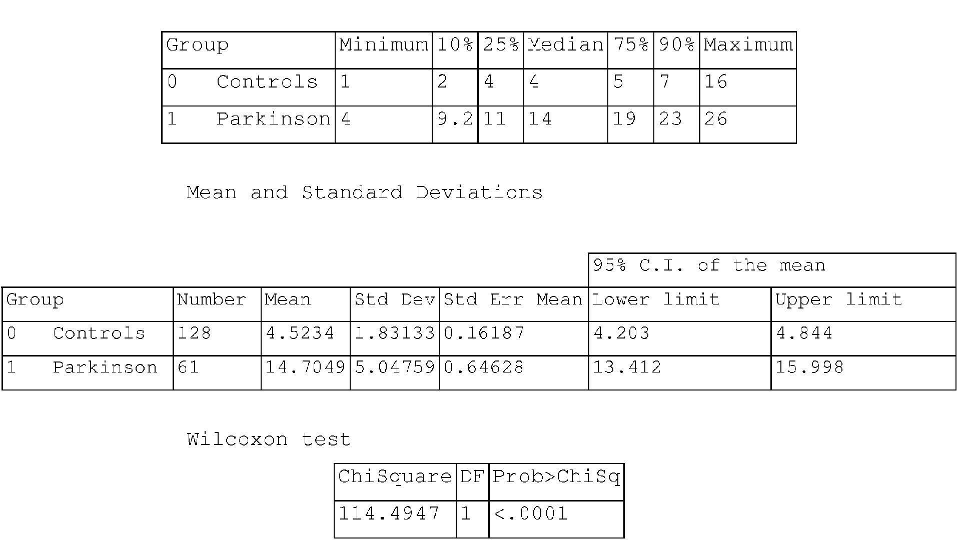

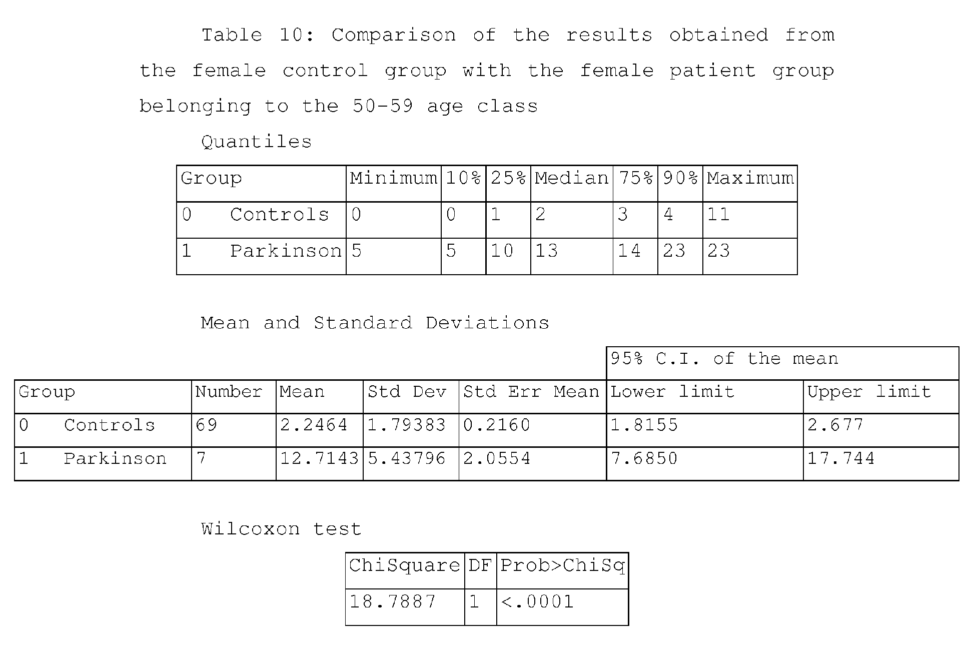

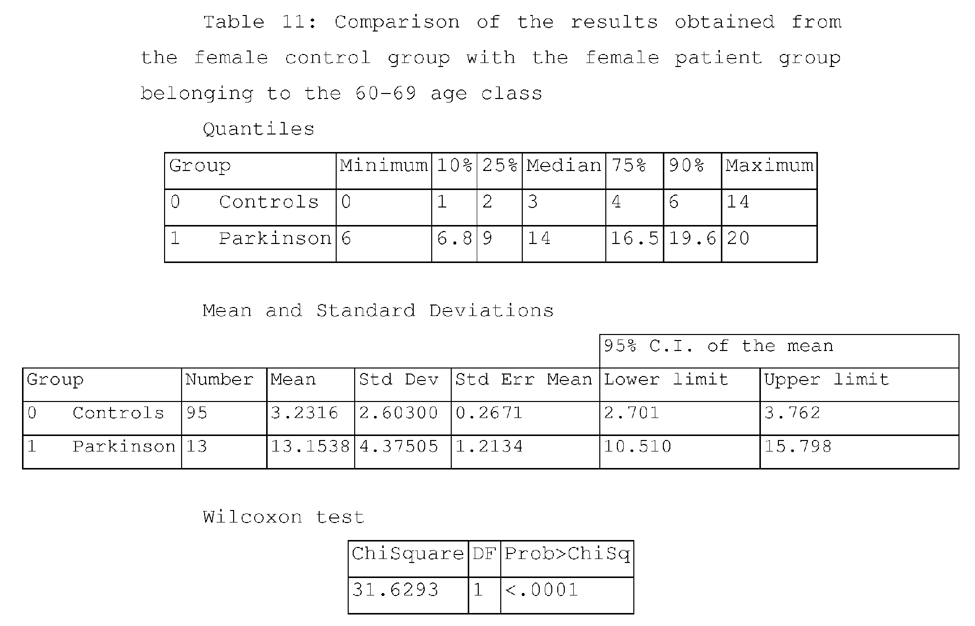

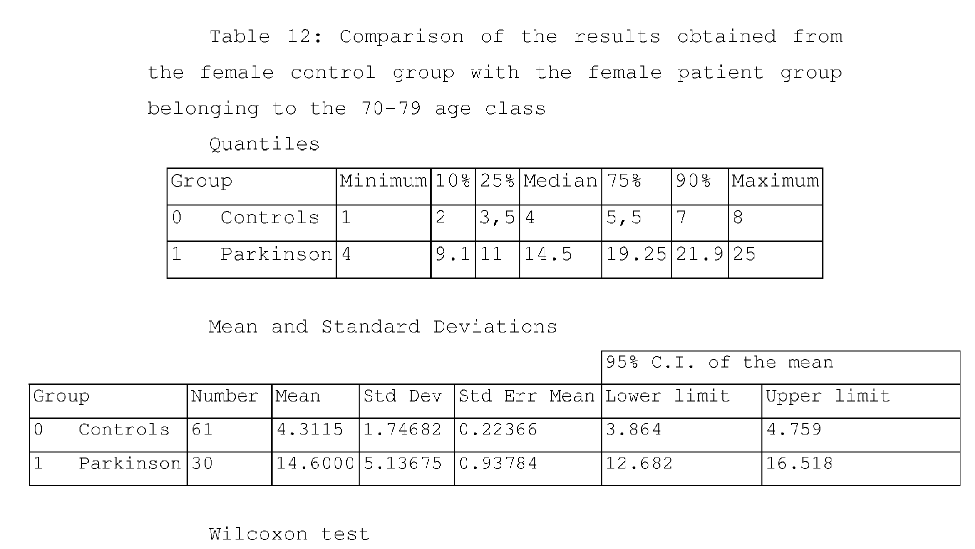

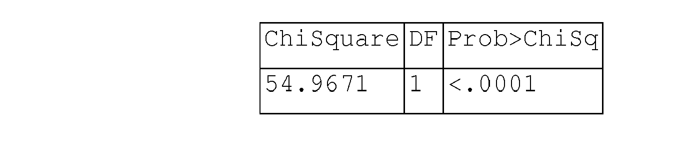

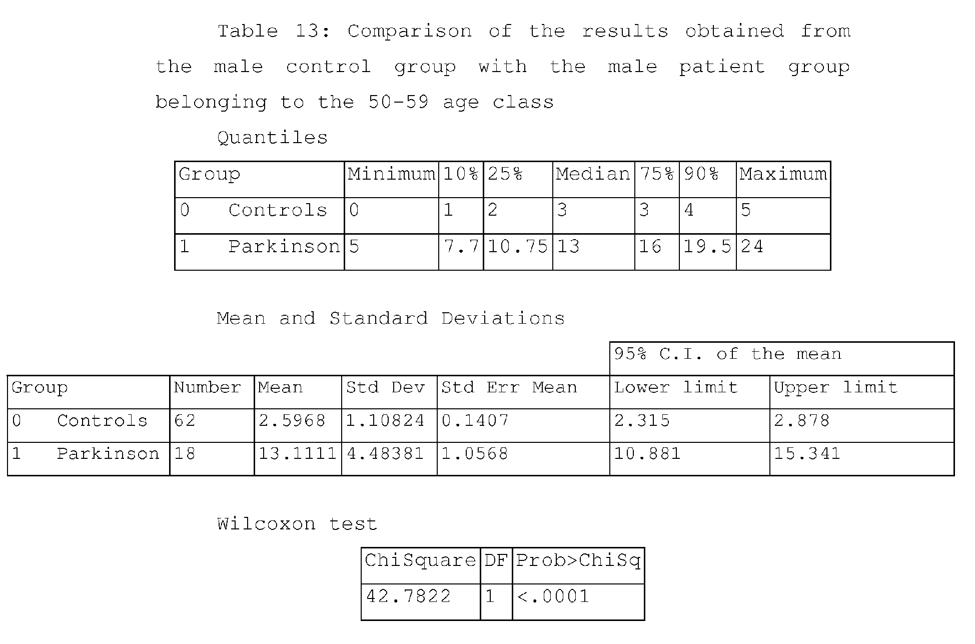

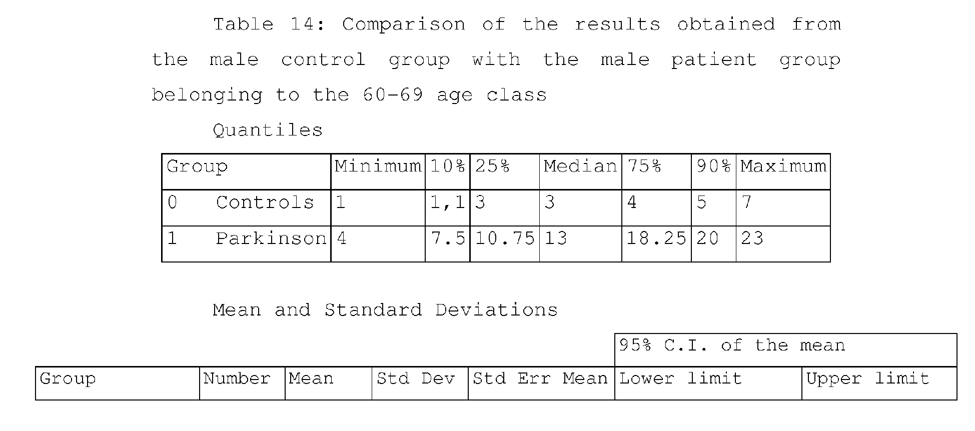

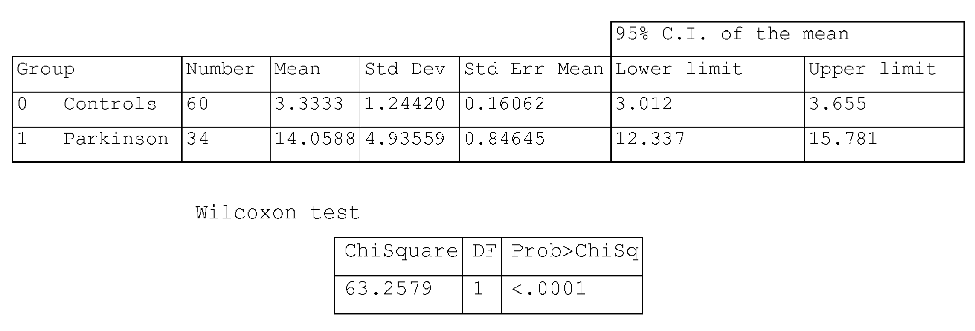

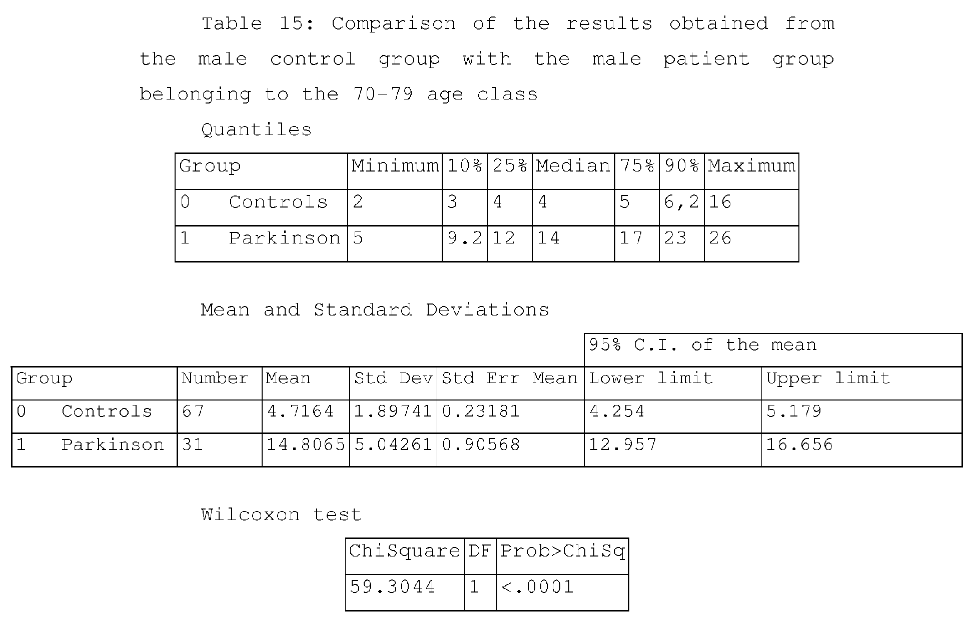

As shown from the results achieved and reported in the present Example, the kit of the invention was found to be effective in detecting olfactory impairments in Parkinson's disease patients in all age classes under examination.

### Example 4: Determining reference values

Test score reference values for different age classes and sexes, expressed as a maximum number of errors in test response, above which the scores are to be deemed pathological and indicative of hypofunction of the sensory system, have been determined using the method that will be referred to as no. 1: values determined from scores found in control subjects for different age classes and sexes, considering as a limit the mean + twice the standard deviation of the number of errors in test response.

In the age classes where olfactory test results obtained from pathological (Parkinson) subjects were also available (50-59; 60-69; 70-79 anni), test reference values were also determined with the method that will be referred to as no. 2:( using the ROC curve).

Therefore, in the 30-39 and 40-49 age classes, for which olfactory test results have been collected from control subjects only (idiopathic Parkinson's disease being poorly represented in those classes), the method 1 was used, considering as a reference the value corresponding to the mean + twice the standard deviation, of the number of errors made by the control subjects in those age classes.

Conversely, for the 50-59; 60-69; 70-79 classes, the reference value was calculated using both methods mentioned above: 1) considering as a reference the value corresponding to the mean + twice the standard deviation, calculated using the scores recorded in the group of control subjects of that age class and sex; 2) using the ROC curve, calculated using the test results obtained both from the control subjects of that age class and sex and those collected in Parkinson's disease patients of the same age class and sex; in this case, the reference value was determined by selecting the value of the number of errors having both the highest specificity and the highest sensitivity.

The numbers of errors in test response above the reference values will be thus considered as pathological values in test response, i.e. values indicative of a hypofunction of the sensory system.

It should be noted that 5 extreme scores of control subjects (4 women and 1 man) were excluded in reference value calculation.

### Results and conclusions:

### Reference limits

The following Table 16 shows the reference limits calculated for male and female subjects and all age classes under examination.

In the 50-59, 60-69 and 70-79 classes, ROC curve reference values are also shown, associated with sensitivity, specificity and Youden index values. Based on the available data, the reference values determined using the ROC curve (method 2) are very close to those determined using the method 1 and have a good discriminating capacity, as confirmed by their high specificity and sensitivity.

More accurate reference limits will be discriminated when larger samples are available.

Table 16: Reference values ranked by sex and age class

| | | | | | | | Reference limit | | | | | | | | | |
|---|---|---|---|---|---|---|---|---|---|---|---|---|---|---|---|---|
| Age class | No. Controls | | No. Pathol. | | Mean (std. dev.) | | Mean + 2*d.s. | | ROC | | Sensitivity | | Specificity | | Youden I. | |
| | F | M | F | M | F | M | F | M | F | M | F | M | F | M | F | M |
| 30≤ Age <40 | 35 | 33 | | | 1.88 (1.34) | 2.15 (1.17) | 4.6 | 4.5 | | | | | | | | |
| 40≤ Age ≤50 | 60 | 57 | | | 2.13 (1.25) | 2.24 (1.09) | 4.6 | 4.4 | | | | | | | | |
| 50≤ Age <60 | 68 | 62 | 7 | 18 | 2.12 (1.45) | 2.60 (1.11) | 5.0 | 4.8 | 4. 5 | 4.5 | 1.00 | 0.95 | 0.95 | 0.95 | 0.95 | 0.95 |
| 60≤ Age <70 | 92 | 60 | 13 | 34 | 2.90 (1.87) | 3.33 (1.24) | 6.6 | 5.8 | 6. 5 | 6.5 | 0.92 | 0.94 | 0.96 | 0.98 | 0.88 | 0.92 |
| 70≤ Age <80 | 61 | 66 | 30 | 31 | 4.31 (1.75) | 4.54 (1.29) | 7.8 | 7.1 | 7. 5 | 7.5 | 0.93 | 0.93 | 0.98 | 0.97 | 0.90 | 0.90 |

Example 5: consistency of test results, when the test is administered to the same subject twice, at different times Test-retest experiment.

The olfactory test of the present patent was administered to the same subjects a first time (test stage) and a second time (retest stage) after a few months. Result consistency was assessed by statistical analysis. More in detail, two experiments were conducted: A and B.

Experiment A. The test was administered (test stage) by a doctor to a group of 57 subjects (32 women and 25 men) having an average age of 59.9 years old (39-77 range). After six months, the same subjects were administered the test a second time (retest stage). The second administration was carried out by paramedical personnel. The consistency of the scores obtained from the two separate administrations by a doctor and a paramedical worker respectively was assessed using: Linear Regression, Pearson, Spearman and Lin Correlation Coefficient, Intraclass Correlation, Paired Sample t-test and Bland & Altman plot.

Experiment B. A group of subjects other than those of the Experiment A was instructed for self-administration of the test in accordance with the directions of use of the invention. Then, 50 subjects (25 women and 25 men) having an average age of 53.3 years old (25-78 range) self-administered the test (test stage). After three months, the test was administered to the same individuals (retest stage) by a doctor. The consistency of the scores obtained from the two separate administrations by self-administration and a doctor respectively was assessed using: Linear Regression, Pearson, Spearman and Lin Correlation Coefficient, Intraclass Correlation, Paired Sample t-test and Bland & Altman plot.

Kits prepared according to Example 2, comprising sets of 33 testers, were used.

### Experiment A results:

### Consistency of test (doctor) with retest (paramedical pers.)

An excellent consistency was found between the number of olfactory test errors made by healthy subjects upon administration of the kit of the invention by a doctor and that recorded for the same subjects upon a second administration of the kit of the invention (retest) by paramedical personnel (specially instructed about the kit administration procedure).

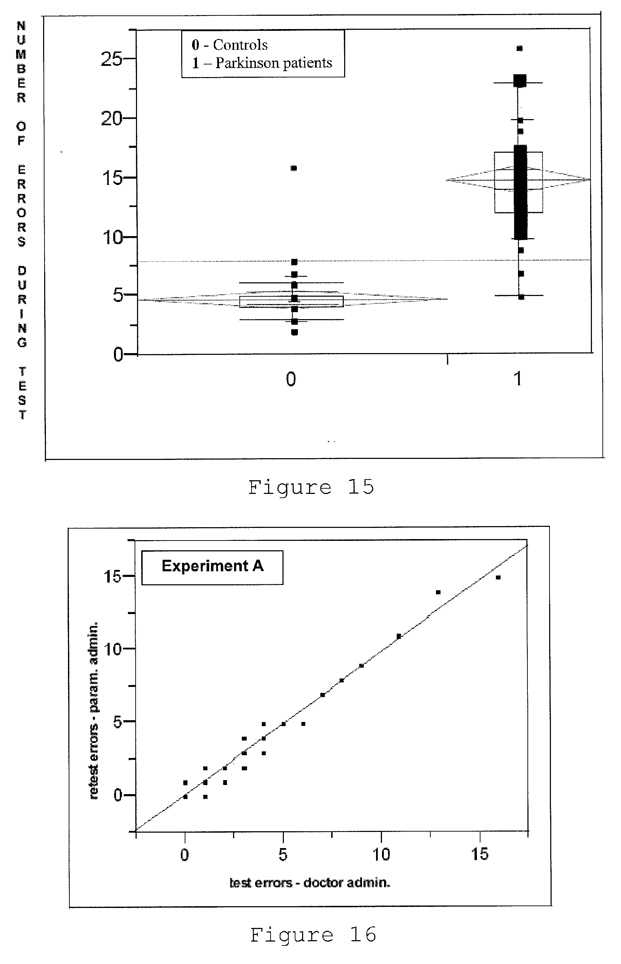

The ratio of the number of errors recorded upon administration of the kit by a doctor to that recorded upon administration by a paramedical worker is shown in Figure 16. The regression coefficient was found to be not significantly different from 1 (p=0.280) and the intercept was found to be not significantly different from 0 (p=0,363). Pearson (r = 0.986) and Spearman (Rho=0.957) Correlation Coefficients were very high. High values were also found for the Lin correlation coefficient (r=0.986, SE=0.004, 95% C.I. = 0.979-0.993) and the Intraclass Correlation Coefficient (r=0.993, 95% C.I. = 0.988-0.996).

No significant difference was found between the number of errors recorded upon administration by a doctor and that recorded upon administration by a paramedical worker (p=0.811) (Tabella 17).

**Table 17**

| Difference between doct_test and param_retest | | | | | | |
|---|---|---|---|---|---|---|
| Difference | | | | limits of 95% consistency | | |
| Mean | | Std. Dev. | | (Bland & Altman. 1986) | | |
| -0.018 | | 0.551 | | -1.097 | 1.062 | |
| Correlation between difference and mean = 0.064 | | | | | | |
| Bradley-Blackwood F = 0.142 (P = 0.86786) | | | | | | |

| Variable | Obs. | | Mean | Std. Dev. | Min. | Max. |
|---|---|---|---|---|---|---|
| Doct_test | 57 | | 3.035088 | 3.33790 | 0 | 16 |
| Param-retest | 57 | | 3.052632 | 3.30271 | 0 | 15 |

### Experiment B results:

### Consistency of test (self-administ.) with retest (doctor administ.)

An excellent concordance was found between the number of errors recorded upon self-administration and that recorded upon administration by a doctor.

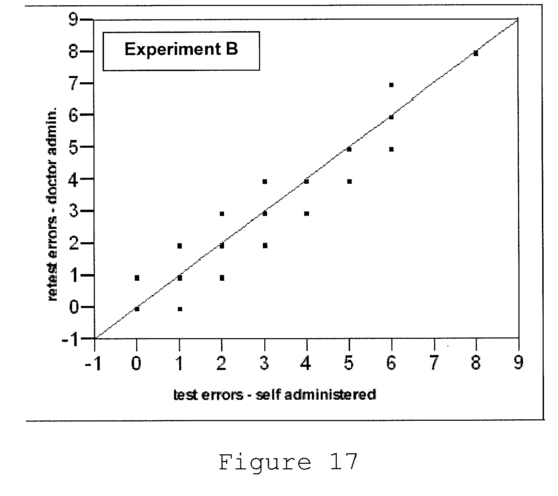

The ratio of the number of errors recorded upon self-administration and that recorded upon administration by a doctor is shown in Figure 17. The regression coefficient was found to be not significantly different from 1 (p=0.960) and the intercept was found to be not significantly different from 0 (p=0,749). Pearson (r = 0.960) and Spearman (Rho=0.959) correlation coefficients were very high. High values were also found for the Lin correlation coefficient (r=0.959, SE=0.011, 95% C.I. = 0.936-0.981) and the Intraclass Correlation Coefficient (r=0.979, 95% C.I. = 0.964-988).

No significant difference was found between the number of errors recorded upon administration by a doctor and that recorded upon administration by a paramedical worker (p=0.622) (Table 18).[SP1]

**Table 18**

| Difference between self_test and doct_retest | | | | | | |
|---|---|---|---|---|---|---|
| Difference | | | | limits of 95% consistency | | |
| Mean | | Std. Dev. | | (Bland & Altman. 1986) | | |
| -0.040 | | 0.570 | | -1.157 | 1.077 | |
| Correlation between difference and mean = -0.139 | | | | | | |
| Bradley-Blackwood F = 0.593 (P = 0.55667) | | | | | | |

| Variable | Obs. | | Mean | Std. Dev. | Min. | Max. |
|---|---|---|---|---|---|---|
| Self_test | 50 | | 2.66 | 1.954691 | 0 | 8 |
| Doct_ retest | 50 | | 2.7 | 2.032893 | 0 | 8 |

The above examples and the results achieved and showed thereby clearly show that the kit of the present invention has a high sensitivity in detecting olfactory identification impairments in Parkinson's disease patients. The test was also found to be reliable in test-retest frameworks and, due to its easy use, to allow administration not only by doctors but also by instructed paramedical personnel, as well as self-administration, by simply following the directions for use provided in the kit. Finally, such kit is conveniently disposable, practical and handy, and is further suitable for mass screening.

### References

From 1 to 29 see references in Table 1.
30. Folstein MF, Folstein SE, MacHug PR. "Mini Mental Satate": a pratical method for grading the cognitive state of patients for the clinician. J Psichiatr Res 1975; 12: 189-198.
31. Murphy C, Schubert CR, Cruickshanks K, Klein BEK, Klein R, Nondahl DM. Prevalence of Olfactory impairment in older adults. JAMA 2002; 13: 2307-12.
32. Panel on Comunicative Disorders to National Advisory Neurological and Comunicative Disorders and Stroke Council. NIH Publication No 79-1914. Washington, Public Health Service, 1979.
33. Gilbert AN, Wysocki CJ. The smell survey results. Natl Geogr Mag 1987; 172-514.
34. Olsson P, Berglind N, Bellander T, Stjärne P. Prevalence of self-reported allergic and non allergic rhinitis symptoms in Stockholm: relation to age, gender, olfactory sense, and smoking. Acta Otolaryngol 2003; 123: 75-80.
35. Landis BN, Konnerth CG, Kummel T. A study on the frequency of olfactory dysfunction. Laryngoscope 2004; 114: 1764-1769.
36. Bramerson A, Johansson L, Ek L, Nordin S, Bende M. Prevalence of olfactory impairment in older adults. JAMA 2002; 288: 2307-2312.
37. Damm M, Temmel A, Welge-Lüssen A, Eckel HE, Kreft MP, Klussmann JP, et al. Epidemiologie und Terapie von Reichstörungen in Deutschland, Ősterreich und Schweiz. HNO 2004; 52: 112-120.
38. Deems DA, Doty RL, Settle RG, Moore-Gillon V, Shaman P, Mester AF, et al. Smell and taste disorders: a study of 750 patients from the University of Pennsylvania Smell and Taste Center. Arch Otorhino laryngol Head Neck Surg. 1991; 117: 519-528.
39. Maremmani C, Del Dotto P, Nocita G, Nassisi V, Rossi G, Bonuccelli U, Muratorio A. Deficit di identificazione di odori in Parkinsoniani "de novo": risultati preliminari. Estratto dagli Atti della 18a Riunione LIMPE, Taormina, 21-23 Novembre 1991. Eds Agnoli A e Battisitn L. Publ D. Guanella, pp 261-268.
40. Nordin S, Monsch AU, Murphy C. Unawareness of smell loss in normal aging and Alzheimer's disease: discrepancy between self-reported and diagnosed smell sensitività. J Gerontol 1995; 50: P187-P192.
41. Muller A, Mungersdorf M, Reichmann H, Srehle G, Hummel T. Olfactory function in Parkinsonian syndromes. J Clin Neurosci 2002; 9: 521-524.
42. Jorgensen MB, Buch NH. Studies on the sense of smell and taste in diabetics. Arch Otolaryngol 1961; 53: 539-545.
43. van Dam FS, Hilgers FJ, Emsbroek G, Touw FI, van As CJ, de Jong N. Deterioration of olfaction and gustation as a consequence of total laryngectomy. Larygoscope 1999; 109: 1150-1155.
44. Frasnelli JA, Temmel AF, Quint C, Oberbauer R, Hummel T. Olfactory function in chronic renal failure. Am J Rhinol 2002; 16: 275-279.
45. Ansari KA, Johnson AJ. Olfactory function in patients with Parkinson's disease. Chronic Dis. 1975; 28: 493-497.
46. Berendse HW, Booij J, Francor CM et al. Subclinical dopaminergic dysfunction in asymptomatic Parkinson's disease patient's relatives with a decreased sense of smell. Ann Neurol 2001; 50: 34-41.
47. Bonuccelli U, Maremmani c, De Dotto P, Nocita G, Rossi G, Muratorio A. Olfactory deficits in Parkinson's disease. Neurology 1992; 42 (Suppl. 3): 440 P990.
48. Braak H, Del Tredici K, Rub U, de Vos RA, Braak E. Staging of the brain pathology related to sporadic Parkinson's disease. Neurobiological Aging 2003; 24: 197-211
49. Crino PB, Martin JA, Hill WD, Greenberg B, Lee VM, Trojanowski JQ. Beta-amyloid peptide and amyloid precursor proteins in olfactory mucosa of patients with Alzheimer's disease, Parkinson's disease and Down syndrome. Ann Otol Rhinol Laryngol 1995; 104: 665-661.
50. Daniel SE, Hawkes CH. Preliminary diagnosis of Parkinson's disease using olfactory bulb pathology. Lancet 1992; 340: 186.
51. Doty RL, Deems DA, Stellar S. Olfactory dysfunction in parkinsonism: a general deficit unrelated to neurologic signs, disease stage, or disease duration. Neurology 1988; 38 (8): 1237-44.
52. Doty RL, Stern MB, Pfeiffer C, Gollomp SM, Hutig HI. Bilateral olfactory dysfunction in early stage treated and untreated idiopathic Parkinson's disease. J Neurol Neurosurg Psychiatry 1992; 55: 138-42.
53. Hawkes C. Olfaction in Neurodegenerative Disorders. Movement Disorders, Vol 18, N° 4, 2003, pp 364 - 372.
54. Hawkes CH, Shephard BC, Daniel SE. Olfaction disorders in Parkinson's disease. J Neurol Neurosurg Psychiatry 1997; 62: 436-46.
55. Maremmani C. Deficit olfattivi nelle malattie neurodegenerative. Tesi di Dottorato di Ricerca. Corso di Dottorato di Ricerca in Discipline Neurologiche e Neurosensoriali. VI ciclo. Anno Accademico 1992 - 1993. Biblioteca della Facoltà di Medicina e Chirurgia, Università di Ancona, Università di Firenze.
56. Markopopulou K, Larsen KW, Wszolek EK et al. Olfactory dysfunction in familial parkinsonism. Neurology 1997; 49: 1262-67.
57. Montgomery EB Jr, Baker KB, Lyons K et al. Abnormal performance on the PD test battery by asymptomatic first-degree relatives. Neurology 1999; 52: 757-62.
58. Pearce RKB, Hawkes CH, Daniel SE. The anterior olfactory nucleus in Parkinson's disease. Mov Disord 1995; 10: 283-287.
59. Quinn NP, Rossor MN, Marsden CD. Olfactory threshold in Parkinson's disease. J Neurol Neurosurg Psychiatry 1987; 50: 88-89.
60. Ross W, Petrovitch H, Abbott RD et al. Association of olfactory dysfunction with risk of future Parkinson's disease. Mov disord 2005; 20 (Suppl 10): Abs 439.
61. Sommer U, Hummel T, Cormann K, et al. Detection of presymptomatic Parkinson's disease: combining smell test, transcranial sonography and SPECT. Mov Disord 2004; 19: 1196-202.
62. Stiasny- Kolster K, Doerr Y, MÖller JC, HÖffken H, Behr TM, Oertel WH, Mayer G. Combination of 'idiopathic' REM sleep behaviour disorder and olfactory dysfunction as possible indicator for α-synucleinopathy demonstrated by dopamine transporter FP-CIT-SPECT. Brain, 2005, 128, 126-137.
63. Tissingh G, Berendse HW, Bergams P et al. Loss of olfaction in de novo and treated Parkinson's disease: possible implication for early diagnosis. Mov Disord 2001; 16: 41-46.
64. Ward CD, Hess WA, Calne DB. Olfactory impairment in Parkinson's disease. Neurology 1988; 33: 943-46.
65. Wenning GK, Shephard B, Hawkes CH, Petruckevitch A, Lees, Quinn NP. Olfactory function in atypical parkinsonian syndromes. Acta Neurol Scand, 1995; 91: 247-250.
66. Mocrieff RW. The chemical senses. Hill, London, 1951
67. Amoore JE. Specific anosmia: a clue to the olfactory code. Nature 1967; 214: 1095.
68. Amoore JE. Specific anosmia and the concept of primary odors. Chem Senses 1977; 2: 267.
69. Amoore JE. Specific anosmia and primary odors. In: Theories of Odor and Odor Measurements. Tanyolac N (Ed). Istambul, 1968: 71.
70. Amoore JE, Forrester LJ. Specific anosmia to trethylamine: the first primary odor. J Chem Ecol 1976; 2: 49.
71. Amoore JE, Forrester LJ, Buttery RG. Specific anosmia to 1-pyrroline: the spermous primary odor. J Chem Ecol 1975; 1: 291.
72. Amoore JE, Forrester LJ, Pelosi P. Specific anosmia to isobutyraldehyde: the malty primary odor. Chem. Senses 1976; 1976; 2: 17.
73. Amoore JE, Pelosi P, Forrester LJ. Specific anosmia to 5-a-androsten-16-en-3-one and w-penta-decalactone: the urinous and musky primary odors. Chem Senses 1977; 2: 401.
74. Pelosi P, Viti R. Specific anosmia to 1-carvone: the minty primary odor. Che Senses 1978; 3: 313-337.
75. Pelosi P, Pisanelli AM. Specific anosmia to 1,8-cineole: the camphor primary odour. Chem Senses 1981; 6: 87-90.
76. Buck L, Axel A. A novel multigene family may encode odorant receptors: a molecular basis for odor recognition. Cell 1991; 65: 175-187.
77. Sumner D. On testing the sense of smell. The Lancet. Saturday 3 November 1962: 895-896.
78. Hummel T, Welge-Lüessen A. Assesment of olfactory function. Hummel T, Welge-Lüessen A (eds): Taste and Smell. An Update. Adv Otorhinolaryngol. Basel, Karger, 2006, vol 63: pp 84-98.
79. Hawkes C, Mussadiq S. Why bother testing the sense of smell? Practical Neurology, 2005; 5: 224-29.
80. Gelb DJ, Oliver E, Gilman S. Diagnostic criteria for Parkinson disease. Arch Neurol 1999;56:33-39.
81. Maremmani C, Ceravolo R, Nocita G, Nuti A, Milanta S, Bonuccelli U. Deficit di identificazione di odori nell'atassia cerebellare idiopatica e nel morbo di Parkinson. Nuova Rivista di Neurologia 1993; 3(6): 223-26.
82. Federico G, Maremmani C, Cinquanta L, Baroncelli GI, Fattori B, Saggese G. Mucus of the human olfactory epithelium contains the insuline-like growth factor-I- system which is altered in some neurodegenerative diseases. Brain Research 1999; 835: 306-14.
83. Hawkes CH. Olfaction in neurodegenerative disorders. Mov Disord 2003; 18: 364-72.
84. Stiasny-Kolster K, Doerr Y, Möller JC, Köffken H, Behr TM, Oertel H, Mayer G. Combination of "idiopathic" REM sleep behaviour disorder and olfactory dysfunction as possible indicator for a-synucleinpathy demonstrated by dopamine transporter FP-CIT-SPECT. Brain 2005; 128: 126-37.
85. Cain WS. Sumner's 'On testing the sense of smell' revisited. Yale Journal of Biology and Medicine 1982; 55: 515-519.
86. Rabin M., Cain WS. Odor recognition: Familiarity, identifiability, and encoding consistency. Journal of Experimental Psychology: Learning, Memory, and Cognition 1984; 10: 316-325.
87. Pfaffmann C. Olfaction and Taste: Proceedings. Rockfeller University Press 1969.
88. Bonuccelli U, Maremmani C, Del Dotto P, Nocita G, Rossi G, MuratorioA. Olfactory deficits in Parkinson's Disease. Neurology 1992; 42(Suppl 3): 440: 990P.
89. Bonuccelli U, Maremmani C, Piccini P, Del Dotto P, Nocita G, Muratorio A. Parkinson's disease and Progressive Supranuclear Palsy: differences in performance on an odor identification test. In: Proceedings, 10th International Symposium on Parkinson's Disease, Tokyo; 1991
90. Doty RL, Stern MB, Pfeiffer C, Gollomp SM, Hurtig HI. Bilateral olfactory dysfunction in early stage treated and untreated idiopathic Parkinson's disease. J Neurol Neurosurg Psychiatr 1992; 55: 138-42.

## Claims

1. A kit comprising a set of 25 to 45 testers and directions for use of the kit, wherein
- each tester comprises a single odorant, other than those of the other testers, adhered to an appropriate odorless substrate common to all testers,
- each tester carries a tester identification mark,
- all testers have the same format, colors, size and texture feel, the only distinctive aspect thereamong being said identification mark, which uniquely corresponds to the single odorant adhered to each tester,
- the area delimited by the odorant adhered on each tester, as well as its color and position on the substrate are common to all testers.

2. A kit as claimed in claim 1, wherein the odorants are selected from the group comprising cloves; rose; lavender; banana; pine; mushroom; talc; mint; coconut; strawberry candy; apple; cheese; watermelon; grass; lilac; sage; licorice; washing soap; wood-like aroma; coffee-coffee liqueur; chocolate-cocoa; oregano; basil; rosemary; garlic; lemon; peach; olibanum; orange; anise-Sambuca; pineapple-pineapple juice; balsamic eucalyptol pastille; unpleasant odor like that of decomposing organic matter; peanuts; butter; apricot; chamomile; smoked charcuterie-speck; onion; car/bike tire rubber; carrot; pear; tangerine; parsley; and bitter almonds.

3. A kit as claimed in claim 1 or 2, wherein at least 12 odorants are selected from the group comprising bitter almonds; apple; apricot; mushroom; car/bike tire rubber; butter; lilac; sage; onion; smoked charcuterie-speck; coffee-coffee liqueur; basil; chamomile; rosemary; balsamic eucalyptol pastille; talc; olibanum; unpleasant odor like that of decomposing organic matter; and lavender.

4. A kit as claimed in any one of claims 1 to 3, wherein the odorant is in microencapsulated form.

5. A kit as claimed in any one of claims 1 to 4, wherein the substrate against which the odorant is adhered is an odorless card or a plate of odorless plastic material.

6. A kit as claimed in claim 5, wherein said substrate is a square or rectangular card, having one surface with the odorant adhered thereto.

7. A kit as claimed in any one of claims 1 to 6, wherein said mark is a number, a letter, a symbol or a combination thereof.

8. A kit as claimed in any one of claims 1 to 7, wherein the directions for use comprise
- information about the use of said kit, and
- a list of item, each item being a set of at least three possible answers to the question: "This smell is or resembles more to?" or other similar questions, each set having the same mark as the tester with which the item is associated, wherein the item number is equal to the tester number, and wherein only one of said at least three possible answers corresponds to the odorant adhered to the tester with which the item is associated.

9. A kit as claimed in claim 8, wherein each item consists of a set of four possible answers.

10. A kit as claimed in any one of claims 1 to 9, wherein each tester and its item are designated by an integer.

11. A kit as claimed in any one of claims 1 to 10, comprising a set of 30 to 40 testers.

12. A kit as claimed in claim 11, comprising a set of 33 testers.

13. A kit as claimed in claim 12, wherein the list includes the following items:
| | | | | | | |
|---|---|---|---|---|---|---|
| 1 | a. | LICORICE | | 18 | a. | WASHING SOAP |
| | b. | CLOVES | | | b. | CHOCOLATE |
| | c. | ONION | | | c. | SMOKED CHARCUTERIE |
| | d. | BANANA | | | d. | ROSEMARY |
| | | | | | | |
| 2 | a. | BASIL | | 19 | a. | PICKLES |
| | b. | ROSE | | | b. | WOOD-LIKE AROMA |
| | c. | GASOLINE | | | c. | SMOKED CHARCUTERIE |
| | d. | ORANGE | | | d. | TEA |
| | | | | | | |
| 3 | a. | CHAMOMILE | | 20 | a. | TOBACCO |
| | b. | COFFEE | | | b. | TEA |
| | c. | SMOKED CHARCUTERIE - SPECK | | | c. | UNPLEASANT, SOLPHUROUS ROTTEN EGG ODOR |
| | d. | LAVENDER | | | d. | COFFEE-COFFEE LIQUEUR |
| | | | | | | |
| 4 | a. | CHEESE | | 21 | a. | TEA |
| | b. | GASOLINE | | | b. | PUTRID, SULPHUROUS ODOR |
| | c. | BANANA | | | c. | CHOCOLATE - COCOA |
| | d. | BUTTER | | | d. | CHAMOMILE |
| | | | | | | |
| 5 | a. | PINE | | 22 | a. | GARLIC |
| | b. | COFFEE | | | b. | ORANGE |
| | c. | MIMOSA FLOWERS | | | c. | OREGANO |
| | d. | ORANGE | | | d. | VANILLA |
| | | | | | | |
| 6 | a. | CIGARETTE SMOKE | | 23 | a. | ORANGE |
| | b. | CHAMOMILE | | | b. | BASIL |
| | c. | FRUIT CANDY | | | c. | ONION |
| | d. | MUSHROOM | | | d. | VANILLA |
| | | | | | | |
| 7 | a. | MINT | | 24 | a. | ROSEMARY |
| | b. | COFFEE | | | b. | SMOKED CHARCUTERIE - SPECK |
| | c. | TALC | | | c. | BREAD |
| | d. | FRUIT CANDY | | | d. | PEACH |
| | | | | | | |
| 8 | a. | PICKLES | | 25 | a. | MINT |
| | b. | MINT | | | b. | GASOLINE |
| | c. | COFFEE | | | c. | TOBACCO - CIGARETTE |
| | d. | WATERMELON | | | d. | GARLIC |
| | | | | | | |
| 9 | a. | SAGE | | 26 | a. | MOLD |
| | b. | COCONUT | | | b. | LEMON |
| | c. | GASOLINE | | | c. | VANILLA |
| | d. | WATERMELON | | | d. | PICKLES |
| | | | | | | |
| 10 | a. | PEANUTS | | 27 | a. | PEACH |
| | b. | STRAWBERRY CANDY | | | b. | MOLD |
| | c. | GASOLINE | | | c. | TOBACCO - CIGARETTE |
| | d. | SMOKED CHARCUTERIE | | | d. | FUEL OIL |
| | | | | | | |
| 11 | a. | ORANGE | | 28 | a. | WATERMELON |
| | b. | CHOCOLATE | | | b. | SMOKED CHARCUTERIE - SPECK |
| | c. | PUTRID, SULPHUROUS ODOR | | | c. | OLIBANUM |
| | d. | APPLE - APPLE | | | d. | ORANGE |
| | | SHAMPOO | | | | |
| | | | | | | |
| 12 | a. | ONION | | 29 | a. | KITCHEN GAS |
| | b. | FUEL OIL | | | b. | ORANGE - ORANGE CANDY |
| | c. | CHEESE AROMA | | | c. | MOLD |
| | d. | ACETONE | | | d. | GASOLINE |
| | | | | | | |
| 13 | a. | SMOKED CHARCUTERIE | | 30 | a. | PINEAPPLE |
| | b. | WATERMELON | | | b. | CHAMOMILE |
| | c. | CHOCOLATE | | | c. | ANISE - SAMBUCA |
| | d. | BASIL | | | d. | PUTRID, SULPHUROUS ODOR |
| | | | | | | |
| 14 | a. | GRASS | | 31 | a. | CIGARETTE SMOKE |
| | b. | VANILLA | | | b. | KITCHEN GAS |
| | c. | ROSEMARY | | | c. | PINEAPPLE - PINEAPPLE JUICE |
| | d. | GASOLINE | | | d. | CHAMOMILE |
| | | | | | | |
| 15 | a. | CHOCOLATE | | 32 | a. | BALSAMIC PASTILLE |
| | b. | LILAC AROMA | | | b. | TOBACCO - CIGARETTE |
| | c. | SMOKED CHARCUTERIE | | | c. | PICKLES |
| | d. | COFFEE | | | d. | PUTRID, SULPHUROUS ODOR |
| | | | | | | |
| 16 | a. | SAGE | | 33 | a. | VANILLA - CREAM |
| | b. | CHAMOMILE | | | b. | APRICOT |
| | c. | SMOKED CHARCUTERIE | | | c. | PEAR |
| | d. | GARLIC | | | d. | UNPLEASANT ODOR - like that of decomposing organic matter |
| | | | | | | |
| 17 | a. | PUTRID ODOR - ROTTEN EGGS | | | | |
| | b. | LICORICE | | | | |
| | c. | KITCHEN GAS | | | | |
| | d. | COFFEE | | | | |

14. A kit as claimed in any one of claims 8 to 13, wherein said directions for use of the kit include the following:
(a) Take the tester marked as the first one among those in the kit;
(b) read the answers provided in the item with the same mark as the tester;
(c) using a piece of paper, rub the odorant on the tester;
(d) bring the rubbed odorant on the tester to your nose, about 1 cm from your nostrils, and smell while avoiding any contact between the tester and the nostrils; while sniffing, read again and think about the answers provided for the item;
(e) select one answer (the answer that is believed to be the right one) to the question: "This smell is or resembles more to?";
(f) without waiting too much between one item and the other, but not too quickly, take the tester marked as the second one among those in the kit;
(g) repeat the steps from (b) to (e) for the tester and item marked as the second ones and then for each tester in the kit, following the order of the marks on the testers and items and always using a new piece of paper to rub each tester.

15. A kit as claimed in any one of claims 1 to 14, wherein the directions for use of the kit are in the form of written text, images, pictures, videos or a combination thereof.

16. A kit as claimed in any one of claims 1 to 15, wherein the directions for use of the kit are provided in paper form or on optical media or through remote consultation, or a combination thereof.

17. Use of testers for the preparation of a kit as claimed in any one of claims 1 to 16, for collecting olfactory response data to be employed in the diagnosis of neurological diseases.

18. A method for collection and assessment of olfactory responses in the diagnosis of neurological diseases, said method including the steps of:
a) administering a kit as claimed in any one of claims 1 to 16;
b) collecting the list of answers for each item, when all answers have been given in the same order as provided in the list, according to the directions for use of the kit;
c) comparing the collected answers with the right answers;
d) counting the number of wrong answers in the list of answers and comparing such number with the test reference values, expressed as a maximum number of errors.
